# EUROPÄISCHE PATENTANMELDUNG

(11) **EP 4 059 491 A1**
(43) Veröffentlichungstag der Anmeldung: **21.09.2022**
(21) Anmeldenummer: 21163221.1
(22) Anmeldetag: 17.03.2021
(51) Int. Cl.: A61K 9/127, A61K 9/51, B01J 13/04

(54) **VORRICHTUNG UND VERFAHREN ZUR HERSTELLUNG VON NANOCARRIERN UND/ODER NANOFORMULIERUNGEN**

(71) Anmelder: Evonik Operations GmbH, 45128 Essen (DE)
(72) Erfinder: GOMEZ, Mario, 64297 Darmstadt (DE); LANG, Dr. Jürgen Erwin, 76229 Karlsruhe (DE); ARNDT, Dr. Marcel, 64546 Mörfelden-Walldorf (DE)
(74) Vertreter: Evonik Patent Association

(57) **Zusammenfassung**

Die Erfindung betrifft eine Vorrichtung 0 und Verfahren zur Herstellung von Nanocarriern und/oder Nanoformulierungen sowie entsprechende Verfahrensprodukte. Der Erfindung liegt die Aufgabe zu Grunde, ein alternatives Verfahren zur Herstellung von Nanocarriern bzw. Nanoformulierungen und eine zugehörige Vorrichtung zur Durchführung dieses Verfahrens anzugeben. Die erfindungsgemäße Vorrichtung 0 ist gekennzeichnet durch eine vertikale Ausrichtung der zu einem Wirkelement 3 führenden Zuleitungen 4, 5. Die Zuleitungen 4, 5 sind ineinander geschachtelt und hinsichtlich ihrer Ausrichtung zueinander axial verstellbar. Das erfindungsgemäße Verfahren sieht die Vermischung von mindestens zwei flüssigen Phasen mit unterschiedlicher Acidität vor. Der Volumenstrom der ersten Phase ist größer als der der zweiten Phase.

## Beschreibung

Ein Nanocarrier im Sinne der Erfindung ist ein Mittel, welches zum Tragen von pharmazeutischen, kosmetischen oder nutrazeutischen Wirkstoffen bestimmt ist. Der Nanocarrier kann aus einem reinen Stoff bestehen oder ein Gemisch mehrerer Stoffe sein. Die Stoffe können fest, semi-fluid oder flüssig sein. Die Stoffe können in einer Phase oder mehrphasig vorliegen: In allen Fällen liegen Nanocarrier in partikulärer Form vor, wobei die mittlere Partikelgröße kleiner als 300 nm ist.

Beispiele für Nanocarrier sind Partikel aus natürlichen oder synthetischen Polymeren, Lipide (Lipid Nano Particles - LNP), Liposomen sowie Mizellen und Nanoemulsionen.

Aus mehreren Stoffen bestehende Nanocarrier werden in der Regel dadurch hergestellt, dass die einzelnen Stoffe oder Präkursoren davon als Dispersionen oder als Lösungen in flüssigen Medien bereitgestellt und miteinander vermischt werden. Bei der Mischung kommt es zu physikalischen Wechselwirkungen zwischen den einzelnen Stoffen oder Präkursoren wobei sich der Nanocarrier bildet. Der Nanocarrier kann anschließend ggf. noch weiter aufgearbeitet werden, bspw. durch Abtrennung aus dem flüssigen Medium. Der erhaltene Nanocarrier kann dann entweder als Placebo verwendet werden oder er wird mit einem Wirkstoff beladen, sodass eine Nanoformulierung entsteht.

Eine Nanoformulierung im Sinne dieser Erfindung ist eine Darreichungsform eines pharmazeutischen, kosmetischen oder nutrazeutischen Wirkstoffes, der von einem Nanocarrier getragen wird. Der Wirkstoff kann sich auf der Oberfläche des Nanocarriers befinden, er kann sich im Innern des Nanocarriers befinden oder er kann mit dem Nanocarrier komplexiert sein.

Beispiele für Nanoformulierungen sind u.a. so genannte Lipoplexe oder Polyplexe, das sind Komplexe aus Polymeren oder Lipiden mit zum Beispiel DNA, RNA, Proteine, Peptiden, etc. Innerhalb dieser Komplexe bilden die Polymere bzw. Lipide den Nanocarrier, währenddessen die DNA bzw. RNA den Wirkstoff darstellen.

Die Herstellung von Nanoformulierungen kann wie vorstehend geschildert dadurch erfolgen, dass ein Nanocarrier mit Wirkstoff beladen wird.

In der pharmazeutischen Technologie ist es aber im Interesse der Verfahrensökonomie, dass die Herstellung des Nanocarriers und dessen Beladung mit Wirkstoff in einem integrierten Prozess erfolgt:
Bei einem solchen integrierten Prozess wird der Nanocarrier erst hergestellt und in status nascendi mit Wirkstoff beladen. Dies erfolgt in der Regel dadurch, dass die einzelnen Komponenten der Nanoformulierung als Dispersion oder Lösung in flüssigen Medien bereitgestellt und vermischt werden. Zu den Komponenten zählen die Stoffe, die den Nanocarrier bilden bzw. Präkursoren dieser Stoffe, sowie der Wirkstoff oder dessen Präkursoren. Bei der Mischung kommt es zu physikalischen Wechselwirkungen zwischen den einzelnen Komponenten, wobei sich die Nanoformulierung bildet. Die Nanoformulierung wird anschießend erforderlichenfalls weiter aufgearbeitet, wie etwa aus dem flüssigen Medium abgetrennt.

Insbesondere Nanoformulierungen, die als Carrier LNP oder Polyplexe verwenden, werden stets in einem integrierten Prozess hergestellt, bei dem die Beladung des Carriers mit Wirkstoff *in statu nascendi* erfolgt.

Ein wesentlicher apparatetechnischer Aspekt bei der Herstellung von Nanocarriern bzw. Nanoformulierungen ist das Design der Mischer, mit dem die in den flüssigen Medien dispergierten oder gelösten Komponenten vermischt werden.

Bei herkömmlichen Mischern ist das Problem das Vermischen selbst. Deshalb werden die Mischer klein strukturiert, so genannte Mikromischer. In einem solchen Mikromischer wird der Massenfluss in viele kleine Teilströme geteilt und die Teilströme gemischt. Diese kleinen Strukturen sind sehr empfindlich hinsichtlich Fouling (Ablagerungen auf der Wand) und kleiner Gasblasen. Deshalb müssen die Edukte für Mikromischer auf Hochreinbedingungen konditioniert werden. Dies bedeutet, dass insbesondere gelöste Gase wie Luft oder Stickstoff aufwendig aus der Flüssigkeit entfernt werden müssen.

Aus EP1519714B1 ist ein Verfahren zur Herstellung von Nanoformulierungen bekannt, bei dem eine Vorrichtung mit einem T-förmigen Mischer verwendet wird. Die beiden zu mischenden Fluide werden durch koaxiale Zuleitungen auf einen Kollisionspunkt geführt, dort vermischt und durch einen um 90° versetzen Ablauf abgezogen. Der Winkel zwischen den beiden Zuleitungen beträgt 180°. Der Volumenstrom beider Fluide in das Wirkelement ist gleich.

Aus EP1937213B1 ist ein Verfahren und eine Vorrichtung zur Herstellung von Nanoformulierungen bekannt, bei dem zwei T-förmige Mischer hintereinander geschaltet sind. Mithin können drei Fluide in zwei Schritten vermischt werden. Nachteil dieser Konstellation ist, dass sich in T-förmigen Mischern komplexe Strömungsverhältnisse ausbilden, die sich beim Vergrößern des Produktionsmaßstabes - dem so genannten "up scaling" - verändern (strömungsmechanisches Ähnlichkeitsproblem). Die veränderten Strömungsverhältnisse bewirken veränderte Produkteigenschaften. Aus diesem Grunde ist es nicht ohne weiteres möglich, die im Labor- und Pilotbetrieb hinsichtlich Produktqualität optimierten Betriebsbedingungen in einen größeren Produktionsmaßstab zu übernehmen. Stattdessen wird im industriellen Betrieb eine Vielzahl von kleinformatigen Apparaten mit den im Pilotmaßstab optimierten Bedingungen parallel betrieben ("numbering up"). Dies erhöht die Investitionskosten. Ein Weiterer Nachteil dieses Verfahrens ist, dass bei der Produktion von LNP ein Gemisch entsteht, in dessen Milieu die Nanocarrier nicht lange stabil sind. Folglich müssen die Nanocarrier entweder besonders schnell aus dem Gemisch abgetrennt werden oder in einem zusätzlichen Schritt stabilisiert werden. Beides macht die Verfahrensführung sehr aufwändig.

Aus der EP3711749A1 sind Y-förmige Mischer zur Herstellung von Nanoformulierungen bekannt. Die beiden Zuleitungen auf den Kollisionspunkt verlaufen mithin nicht parallel. Der Y-Mischer kann mit einem T-Mischer kombiniert sein, um dreiphasige Gemische herzustellen. Y-Mischer weisen ebenfalls eine komplexe Strömungsdynamik auf, weswegen sie nur im Wege des "numbering up" aus dem Pilot- in den Produktionsmaßstab überführt werden können. Das Problem der mangelnden Stabilität von LNP in dem erhaltenen Gemisch besteht bei Prozessen mit Y-Mischern auch.

Aus der WO2001005373A1 ist ein Mischkopf zur Herstellung von Nanoformulierungen bekannt, der mehrere Injektoren mit einem Durchmesser von weniger als 2 mm aufweist.

WO2017223135A1 offenbart einen Mischkopf, der ebenfalls nach dem Injektor-Prinzip arbeitet. Der Injektor ist mit einer Dosiervorrichtung versehen, die als eine servomotorisch angetriebene Pipette ausgeführt ist.

Der Erfindung liegt die Aufgabe zu Grunde, eine Vorrichtung zur Herstellung von Nanocarriern bzw. Nanoformulierungen anzugeben, deren strömungsmechanischen Verhältnisse auch bei einem "scale up" erhalten bleiben, sodass eine Vergrößerung des Produktionsmaßstabes ohne ein "numbering up" möglich ist.

Darüber hinaus soll das mit der Vorrichtung betriebene Verfahren zur Herstellung von Nanocarriern bzw. Nanoformulierungen Arbeitsschritte einsparen.

Gelöst wird die Aufgabe durch eine Vorrichtung nach Anspruch 1 und durch ein vermittels dieser Vorrichtung durchführbares Verfahren nach Anspruch 13.

Gegenstand der Erfindung ist mithin eine Vorrichtung für die Herstellung von Nanocarriern und/oder Nanoformulierungen, welche die folgenden Merkmale umfasst:
- ein erstes Vorlagegefäß zur Aufnahme einer ersten flüssigen Phase;
- ein zweites Vorlagegefäß zur Aufnahme einer zweiten flüssigen Phase;
- ein Wirkelement zum Bereitstellen einer zumindest zweiphasigen Mischung durch Mischen der ersten flüssigen Phase mit der zweiten flüssigen Phase;
- einen ersten Zulauf, über den das erste Vorlagegefäß mit dem Wirkelement fluidleitend verbunden ist;
- einen zweiten Zulauf, über den das zweite Vorlagegefäß mit dem Wirkelement fluidleitend verbunden ist;
- ein Sammelgefäß zur Aufnahme der Mischung;
- einen Ablauf, über den das Wirkelement mit dem Sammelgefäß fluidleitend verbunden ist;
- eine Pumpe, die in den Ablauf dergestalt eingegliedert ist, dass die Mischung vermittels der Pumpe von dem Wirkelement über den Ablauf in das Sammelgefäß förderbar ist.

Erfindungsgemäß ist bei der Vorrichtung:
- dass der erste Zulauf und der zweite Zulauf zumindest auf einem Vertikalabschnitt vertikal ausgerichtet sind;
- dass auf dem Vertikalabschnitt der zweite Zulauf innerhalb des ersten Zulaufs geführt ist;
- und dass auf dem Vertikalabschnitt erste Zulauf oder der zweite Zulauf oder beide Zuläufe axial verschiebbar ausgeführt sind, dergestalt, dass die axiale Position des zweiten Zulaufs gegenüber dem ersten Zulauf veränderlich ist.

Die erfindungsgemäße Vorrichtung ist gekennzeichnet durch eine vertikale Ausrichtung der zum Wirkelement führenden Zuläufe: Der erste und der zweite Zulauf sind beide zumindest auf einem Abschnitt stromaufwärts vor dem Wirkelement vertikal angeordnet, sodass die Strömung durch die Zuläufe in Richtung der Erdbeschleunigung ausgerichtet sind. Der Abschnitt, in dem die Zuläufe vertikal verlaufen, wird hier Vertikalabschnitt genannt. Dies ist kein eigenes Bauelement der Vorrichtung, sondern vielmehr ein Bereich der Vorrichtung, in dem die Zuläufe sich vertikal erstrecken. Da die Strömungsrichtung durch den Vertikalabschnitt parallel zu der Erdbeschleunigung ist - also nach unten - und das Wirkelement sich stromabwärts seiner Zuläufe befindet, erstreckt sich der Vertikalabschnitt denknotwendig oberhalb des Wirkelements. Allerdings muss der Vertikalabschnitt nicht lotrecht über dem Wirkelement liegen, denn die Zuläufe können außerhalb des Vertikalabschnitts auch horizontal oder geneigt verlaufen. Allerdings liegt der Vertikalabschnitt vorzugsweise lotrecht oberhalb des Wirkelements; besonders bevorzugt erstreckt sich der Vertikalabschnitt bis zum dem Wirkelement, sodass die flüssigen Phasen vertikal in das Wirkelement einlaufen. Bei dieser Konstellation sind die Strömungsvektoren parallel zu dem Gravitationsvektor ausgerichtet.

Ein weiteres wesentliches Merkmal der erfindungsgemäßen Vorrichtung ist, dass der zweite Zulauf auf dem Vertikalabschnitt innerhalb des ersten Zulaufes geführt ist. Dies bedeutet, dass der zweite Zulauf von dem ersten Zulauf umgeben ist. Die Wandung des zweiten Zulaufes steht folglich innen mit der zweiten flüssigen Phase und außen mit der ersten flüssigen Phase in Kontakt. Die Wandung des ersten Zulaufs kontaktiert innen die erste flüssige Phase und außen die Umgebung.

Die vertikale Ausrichtung der Zuläufe und ihre geschachtelte Anordnung wirken sich positiv auf die Strömungsverhältnisse im Wirkelement aus, was zu einer besonderes homogenen Vermischung der Phasen führt. Außerdem haben sich die durch diese Gestaltungsweise hervorgerufenen Strömungsverhältnisse als besonders stabil erwiesen, wenn die Strömungsquerschnitte variiert werden. Bei kleinen Strömungsquerschnitten optimierte Betriebsbedingungen werden daher auch bei einem scale up beibehalten.

Prinzipiell erzeugt die Vorrichtung mit ihrem Wirkelement fluiddynamisch einen dünnen Film für den mischenden Stoffaustausch. Hierbei ist das Verhältnis von Kapillarströmung und Randströmung des Puffermediums relevant. Der erzeugte dünne Film (Teleskop-Antenneneffekt) mischt sich im Wesentlichen durch Diffusion, ohne dass Fouling und Verblockung an der Grenzfläche auftreten. Da die Ströme bei der erfindungsgemäßen Vorrichtung nicht durch Mikrostrukturen "gedrückt" werden müssen, verläuft der Vorgang nahezu drucklos, weshalb die Anordnung und das Verfahren einfach vergrößert (scale up) werden kann.

Ein weiteres Gestaltelement der Erfindung sieht vor, dass der erste und/oder der zweite Zulauf auf dem Vertikalabschnitt axial verschieblich ausgeführt sind, sodass sich die axiale Position der beiden Zuläufe relativ zueinander verändern lässt. Über die Variation der Axialausrichtung können die Strömungsverhältnisse innerhalb des Wirkelements verstellt werden, sodass das Mischergebnis optimiert werden kann.

Vorzugsweise ist die Pumpe der Vorrichtung als eine Kreiselpumpe ausgeführt ist, welche ein Gehäuse und ein im Gehäuse um eine Drehachse drehbar gelagerten Läufer (Pumpenrad) aufweist, der mit einem Antrieb drehend antreibbar ist. Der Antrieb ist einfachstenfalls ein Motor der mechanisch über eine Welle an den Läufer der Pumpe gekuppelt ist. Eine bevorzugte Variante sieht eine magnetische Kraftübertragung ohne mechanische Welle vor. In beiden Fällen ist die Drehachse des Läufers vorzugsweise vertikal angeordnet.

Die Tatsache, dass die Drehachse der Kreiselpumpe vertikal verläuft, bedeutet, dass die Kreiselpumpe das Gemisch in der Horizontalebene beschleunigt. Da die flüssigen Phasen auf ihrem Weg durch den Vertikalabschnitt in Fallrichtung fließen und dann nach dem Mischen horizontal beschleunigt werden, bedeutet, dass die Flüssigkeiten bei ihrem Fließweg durch die Zuläufe, das Wirkelement und die Pumpe um 90° umgelenkt werden. Dies intensiviert die Vermischung. Die Kreiselpumpe übernimmt somit auch einen Teil der Mischaufgabe, sie stellt quasi eine zweite Mischstufe dar. Bei einer vertikalen Anordnung der Drehachse lässt sich der Läufer außerdem ein wenig destabilisieren und sich die Pumpe so auch zum Quenchen nutzen. Zudem kann bei einer koaxialen Ausrichtung der Drehachse zur Achse des Vertikalabschnitts die Mischung in Regionen des Gehäuses einströmen, wo sie via Wandströmung weitergefördert werden. Dies wirkt sich auch positiv auf die Produktbildung aus.

All diese erfindungsgemäßen Gestaltelemente resultieren in einer besonders intensiven Vermischung der flüssigen Phasen, sodass die darin enthaltenen Komponenten besonders eng miteinander in Kontakt geraten und effizient wechselwirken können. Die durch die großen Geschwindigkeitsunterschiede hervorgerufenen, intensiven Diffusionsvorgänge sorgen für eine Feinverteilung der nanoskaligen Stoffe in dem Dispersionsmedium. Das Ergebnis ist ein Gemisch, in dem der Nanocarrier bzw. die Nanoformulierung eine besonders enge Partikelgrößenverteilung aufweist.

Ein weiterer Vorteil der erfindungsgemäßen Vorrichtung ist ihr einfacher und robuster Aufbau. Dies erhöht die Betriebssicherheit. Außerdem lässt sich die Vorrichtung sowohl Batchweise als auch kontinuierlich betreiben. Damit kann das Herstellverfahren auf derselben Vorrichtung zunächst in einem Batchbetrieb optimiert werden und sodann einfach durch eine Umstellung auf kontinuierlichen Betrieb der Produktionsdurchsatz angehoben werden. Darüber hinaus haben sich die Strömungsverhältnisse in dem Wirkelement als besonders stabil erwiesen, sodass sich beim Vergrößern der Durchmesser im Zuge einer Kapazitätserhöhung kaum Ähnlichkeitseffekte bemerkbar machen, welche die Produktqualität beeinträchtigen. Folglich lässt sich mit der erfindungsgemäßen Vorrichtung ein echtes "scale up" durchführen, um die Kapazität zu erweitern. Dies erlaubt eine besonders wirtschaftliche Übertragung der im Versuchsbetrieb gewonnenen Erkenntnisse auf den industriellen Produktionsmaßstab.

Im Ergebnis können mit der erfindungsgemäßen Vorrichtung kostengünstig Verfahren entwickelt und dann mit gleichbleibend hoher Qualität produziert werden. Die Erfindung ermöglicht daher eine besonders wirtschaftliche Produktion.

Ein weiterer Vorteil der erfindungsgemäßen Vorrichtung ist, dass ihr Wirkelement ohne mikrostrukturierte Bauteile auskommt und damit weniger anfällig für Fouling und Blockagen ist.

Eine bevorzugte Weiterbildung der Vorrichtung sieht vor, dass der erste Zulauf innerhalb des Vertikalabschnitts aus einer ersten linearen Rohrleitung gebildet wird, dass der zweite Zulauf innerhalb des Vertikalabschnitts aus einer zweiten linearen Rohrleitung gebildet wird, und dass die erste lineare Rohrleitung und die zweite lineare Rohrleitung auf dem Vertikalabschnitt koaxial verlaufen. Die Zuleitungen sind in dieser Ausführungsform zumindest auf dem Vertikalabschnitt als feste Rohrleitungen ausgebildet und verlaufen koaxial. Die festen Rohrleitungen ermöglichen besonders stabile Strömungsverhältnisse und eine exakte axiale Positionierbarkeit. Außerhalb des Vertikalabschnitts können die Zuleitungen auch flexibel ausgeführt sein, etwa als Schlauchleitung. Dies kann im Interesse der axialen Verschiebbarkeit auch notwendig sein. Die Koaxialität, also die mittige Anordnung der zweiten Zuleitung in der ersten Zuleitung, ermöglicht ein besonders stabiles Strömungsprofil im Wirkelement.

Vorzugsweise ist die zweite lineare Rohrleitung als eine Kapillare mit einem gegenüber dem Querschnitt der ersten linearen Rohrleitung viel kleinerem Querschnitt ausgeführt. Grund dafür ist, dass die Durchflussmenge (Volumenstrom) durch den ersten Zulauf erfindungsgemäß viel größer ist als die Zugabemenge der zweiten flüssigen Phase. Die erste und die zweite lineare Rohrleitung haben vorzugsweise beide einen kreisrunden Querschnitt. Es ist auch möglich, die zweite Zuleitung zweiadrig auszulegen, also aus zwei parallel verlaufenden Rohrleitungen oder Kapillaren, durch welche jeweils der halbe Volumenstrom der zweiten flüssigen Phase fließt.

In ihrer einfachsten Ausführung erlaubt die erfindungsgemäße Vorrichtung lediglich die Herstellung einer zweiphasigen Mischung aus der ersten flüssigen Phase und der zweiten flüssigen Phase (binäres System). Es sind aber auch mehrere Varianten möglich, in denen die Vorrichtung zur Herstellung einer zumindest dreiphasigen Mischung aus einer ersten, zweiten und dritten flüssigen Phase (tertiäres System) ermöglicht.

Ausführungsformen der Vorrichtungen, die lediglich zweiphasige Mischungen erzeugen können, werden hier "binäre Vorrichtung" genannt, währenddessen Vorrichtungen, welche die Herstellung von dreiphasigen Mischungen ermöglichen, als "tertiär" bezeichnet werden.

Jede Variante der tertiären Vorrichtung umfasst notwendigerweise ein drittes Vorlagegefäß zur Aufnahme einer dritten flüssigen Phase sowie einen dritten Zulauf, über den das dritte Vorlagegefäß mit dem Wirkelement fluidleitend verbunden ist, wobei das Wirkelement zum Bereitstellen einer zumindest dreiphasigen Mischung durch Mischen der ersten flüssigen Phase mit der zweiten flüssigen Phase und der dritten flüssigen Phase eingerichtet ist, und wobei der erste Zulauf innerhalb des Vertikalabschnitts aus einer ersten linearen Rohrleitung gebildet ist, der zweite Zulauf innerhalb des Vertikalabschnitts aus einer zweiten linearen Rohrleitung gebildet ist und der dritte Zulauf innerhalb des Vertikalabschnitts aus einer dritten linearen Rohrleitung gebildet ist. Dem allgemeinen Erfindungsgedanken folgend, ist der dritte Zulauf zumindest auf dem Vertikalabschnitt vertikal ausgerichtet, er ist auf dem Vertikalabschnitt innerhalb des ersten Zulaufs geführt, und der der dritte Zulauf ist auf dem Vertikalabschnitt axial verschiebbar ausgeführt, dergestalt, dass die axiale Position des dritten Zulaufs gegenüber dem ersten Zulauf veränderlich ist. Der dritte Zulauf erfüllt mithin dieselben Gestaltmerkmale wie der zweite Zulauf.

Bei einer ersten Variante einer tertiären Vorrichtung verlaufen die zweite Rohrleitung und die dritte Rohrleitung parallel zueinander durch die erste Rohrleitung. Dies bedeutet einfachstenfalls, dass die Wandung der dritten Rohrleitung innen mit der dritten flüssigen Phase in Kontakt kommt und außen mit der ersten flüssigen Phase. (Die Wandung des zweiten Zulaufes steht weiterhin innen mit der zweiten flüssigen Phase und außen mit der ersten flüssigen Phase in Kontakt.) Die zweite und die dritte Rohrleitung sind vorzugsweise jeweils als Kapillare mit geringerem Querschnitt als die äußere Rohrleitung ausgeführt. Bei dieser Konstellation können die Rohrleitungen nicht koaxial ausgerichtet sein. Im Prinzip gleicht diese Bauweise einertertiären Vorrichtung einer binären Vorrichtung mit einer zweiadrigen zweiten Rohrleitung.

In einer zweiten, bevorzugten Varianten einer tertiären Vorrichtung können die zweite Rohrleitung und die dritte Rohrleitung parallel durch die erste Rohrleitung verlaufen. Die drei Rohrleitungen sind dann nicht koaxial. Die erste Rohrleitung kann dann auch mit einem ringförmigen Querschnitt aufweisen: Dieser wird dadurch erreicht, dass die erste Rohrleitung eine separate Innenwandung erhält. Die erste flüssige Phase fließt dann durch den zwischen der Innenwandung und Außenwandung gebildeten Ringspalt. Die erste Rohrleitung erhält dann eine Seele, durch welche keine erste flüssige Phase fließt. Durch die Seele werden dann die zweite und die dritte Rohrleitung geführt. Zwischen der Außenwandung der zweiten und der dritten Rohrleitung sowie der Innenwandung der ersten Rohrleitung bleibt die Seele leer.

In einer dritten Variante einer tertiären Vorrichtung hingegen können alle drei Rohrleitungen auf dem Vertikalabschnitt koaxial verlaufen. Dies ist dadurch möglich, dass der dritte Zulauf auf dem Vertikalabschnitt innerhalb des zweiten Zulaufs geführt ist, währenddessen der zweite Zulauf weiterhin innerhalb des ersten Zulaufes verläuft. Mithin ist die dritte Rohrleitung von der ersten und von der zweiten Rohrleitung umgeben, währenddessen die zweite Rohrleitung nur von der ersten Rohrleitung umgeben ist. Die Wandung der dritten Rohrleitung kontaktiert innen die dritte flüssige Phase und außen die zweite flüssige Phase. Die Wandung der zweiten Rohrleitung kontaktiert innen die zweite flüssige Phase und außen die erste flüssige Phase. Die Wandung der ersten Rohrleitung kontaktiert innen die erste flüssige Phase und außen die Umgebung. Bei dieser dreifach geschachtelten Konstellation können alle drei Rohrleitungen koaxial verlaufen, müssen es aber nicht. Im Interesse einer stabilen Mischung ist aber eine koaxiale Anordnung bevorzugt. Diese dreifach geschachtelte Variante ist apparativ etwas aufwändiger als die erste Variante mit paralleler zweiter und dritter Rohrleitung, erzielt dafür aber eine bessere Homogenität der Mischung.

Gemäß einer bevorzugten Weiterbildung der Erfindung ist die Drehachse des Läufers koaxial zur Achse des ersten Zulaufs im Bereich des Vertikalabschnitts ausgerichtet. Dies bedeutet, dass die Pumpe, genauer gesagt deren Läufer, sich unterhalb des Wirkelements befinden muss. Das aus dem Wirkelement abströmende Gemisch gelangt direkt auf den Läufer und wird dort horizontal nach außen beschleunigt. Der Pumpenläufer dient mithin als zusätzlicher Mischer, der die Komponenten in der Dispersion feinverteilt. Der Ablauf aus der Pumpe ist in dieser Konstellation vorzugsweise zumindest abschnittsweise horizontal auszuführen. Zwischen Zulauf und Ablauf herrscht dann ein rechter Winkel. Innerhalb der Pumpe wird die Strömung um 90° umgelenkt. Diese Konstellation ermöglicht eine Destabilisierung des Läufers, was zu einer geringfügigen Rückförderung führt, die sich positiv auf das Produkt auswirkt.

In einer weiteren bevorzugten Ausführungsform der Vorrichtung ist die Pumpe levitronisch ausgeführt. Dies bedeutet, dass der Läufer im Gehäuse magnetisch gelagert ist und es sich bei dem Antrieb um ein Drehfeld handelt. Das Drehfeld zieht den Läufer der Pumpe mit und ermöglicht so eine Leistungsübertragung von Antrieb auf Läufer frei von mechanischem Kontakt zwischen Antrieb und Läufer. Bei dieser Konstellation ist der Läufer der Pumpe gleichzeitig der Läufer eines Elektromotors; das Gehäuse der Pumpe ist gleichzeitig der Stator des Elektromotors. Pumpe und Antrieb sind integriert. Vorteil dieser Bauweise ist, dass das Gehäuse nicht für eine Antriebswelle durchbrochen werden muss, weil keine Antriebswelle existiert. Statt der mechanischen Leistungsübertragung über eine Antriebswelle erfolgt die Leistungsübertragung bei einer levitronischen Pumpe rein magnetisch über das Drehfeld. Optional kann das Drehfeld auch die Lagerung des Läufers im Gehäuse bewerkstelligen. Auf diese Weise wird nicht nur eine fehleranfällige Dichtung der Antriebswelle eingespart, sondern es ist dem Gemisch möglich beiderseits der Drehebene des Läufers zu strömen, da an keiner Seite eine Antriebswelle den Fluss blockiert. Weiterer Vorteil der levitronischen Kreiselpumpen ist, dass diese so betrieben werden können, dass geringe Kavitationseffekte hervorrufen werden und es so zu weniger Materialabrieb oder-platzungen kommt, welche die Mischung kontaminieren könnten.

Levitronische Pumpen sind kommerziell erhältlich wie zum Beispiel der Pumpentyp PuraLev-i30 der Firma Levitronix GmbH, Zürich, CH.

Ein Vorteil der erfindungsgemäßen Vorrichtung besteht darin, dass sie einfach vom Batch-Betrieb in den kontinuierlichen Betrieb umgebaut werden kann. Dafür ist es lediglich erforderlich, den Ablauf zurück in das erste Vorlagegefäß zu führen, sodass ein Umlauf (Loop) aus dem ersten Vorlagegefäß über den ersten Zulauf in das Wirkelement, durch die Pumpe und zurück in das erste Vorlagegefäß entsteht. Das Sammelgefäß und das erste Vorlagegefäß sind dann identisch, und der Ablauf ist als ein Umlauf ausgeführt. Dies stellt eine besonders bevorzugte Ausführungsform der Erfindung dar. Die Produkte werden bei dieser Konstellation nach einer gewissen Laufzeit der Vorrichtung wahlweise aus dem ersten Vorlagegefäß entnommen oder aus dem Umlauf ausgeschleust. Der Umlauf muss nicht zwangsläufig geschlossen sein: Es ist auch möglich, ein größeres Flüssigkeitsvolumen in der Vorrichtung über den Umlauf zu zirkulieren und gleichzeitig eine kleinere Menge Produkt aus dem Ablauf auszuschleusen. Der entnommenen Menge entsprechend wird dann gleichzeitig frische flüssige Phase in die Vorlagegefäße nachdosiert. Eine solche Verfahrensweise ist eine Mischform von kontinuierlichem Betrieb und Batchbetrieb.

Der Volumenstrom der ersten flüssigen Phase ist in der Regel ist deutlich größer als der der zweiten und/oder dritten Flüssigen Phase. Um den Volumenstrom der ersten flüssigen Phase zu steuern ist es daher ausreichend den Förderstrom der Pumpe zu verändern. Die Volumina der ersten und/oder zweiten flüssigen Phase sind dann vernachlässigbar. Die Vorrichtung weist daher am einfachsten eine Pumpe mit veränderlichem Fördervolumen auf. Das Fördervolumen der Pumpe sollte gesteuert oder geregelt werden. Die Veränderung des Fördervolumens erfolgt am einfachsten über die Drehzahl des Antriebs, also des Drehfelds, das den Läufer Pumpe bzw. des Motors antreibt.

Zur exakten Einstellung der Mischverhältnisse sollte die die Vorrichtung zumindest eine Dosiervorrichtung aufweisen, welche zur Dosierung der zweiten flüssigen Phase in das Wirkelement eingerichtet ist. Da der Volumenstrom der zweiten flüssigen Phase deutlich kleiner ist als der ersten flüssigen Phase, kann die Dosiervorrichtung in einer bevorzugten Variante als eine linear angetriebene Kolbenpumpe ausgeführt sein. Die Kapazität einer Kolbenpumpe ist grundsätzlich durch das Kolbenvolumen begrenzt. Im Batchbetrieb ist dies nicht weiter störend, im kontinuierlichen Betrieb indes schon. Daher sollten im kontinuierlichen Betrieb mehrere Kolbenpumpen vorgesehen sein, die abwechselt dieselbe Phase dosieren. Der eine Kolben wir aufgezogen, währenddessen der andere Kolben dosiert. Gleichwirkend kann eine Kolbenpumpe verwendet werden, die mehrere Kolben umfasst.

Bei einer tertiären Vorrichtung sollte auch eine entsprechende Dosiervorrichtung zur Dosierung der dritten flüssigen Phase in das Wirkelement vorgesehen sein. Hier gilt das zur Dosiervorrichtung für die zweite flüssige Phase Gesagte entsprechend.

Vorzugsweise ist der Förderstrom einer Dosiervorrichtung veränderbar, insbesondere ist der Förderstrom aller Dosiervorrichtungen veränderbar. Bei Kolbenpumpen erfolgt die Veränderung des Volumenstroms über die Veränderung der Geschwindigkeit des Linearantriebs.

Besonders bevorzugt werden die Volumenströme der Kreiselpumpe und der Dosiervorrichtung(en) gemeinsam von einer zentralen Regelungseinheit der Vorrichtung geregelt.

Gegenstand der Erfindung ist auch ein Verfahren zur Herstellung von Nanocarriern und/oder Nanoformulierungen, welches mit Hilfe der hier vorgestellten Vorrichtung durchgeführt wird. Ein erfindungsgemäßes Herstellungsverfahren umfasst die folgenden Schritte:
- Bereitstellen einer erfindungsgemäßen Vorrichtung;
- Bereitstellen einer ersten flüssigen Phase im ersten Vorlagegefäß, wobei die erste flüssige Phase ein erstes flüssiges Dispersionsmedium enthält;
- Bereitstellen einer zweiten flüssigen Phase im zweiten Vorlagegefäß, wobei die zweite flüssige Phase ein zweites flüssiges Dispersionsmedium und mindestens eine Komponente enthält, welche ausgewählt ist aus der Gruppe bestehend aus Präkursor eines Nanocarriers, Präkursor eines Wirkstoffes, Wirkstoff;
- Antreiben der Pumpe zur Etablierung einer Flüssigkeitsströmung aus dem ersten Vorlagegefäß über den ersten Zulauf in das Wirkelement und über den Ablauf in das Sammelgefäß;
- Dosieren der zweiten flüssigen Phase über den zweiten Zulauf in das Wirkelement, wobei der Volumenstrom der zweiten flüssigen Phase in dem zweiten Zulauf kleiner ist als der Volumenstrom der Flüssigkeitsströmung in dem ersten Zulauf;
- Mischen von erster flüssiger Phase und zweiter flüssiger Phase in dem Wirkelement, wobei eine Mischung enthaltend einen Nanocarrier und/oder eine Nanoformulierung erhalten wird;
- Ansammeln der Mischung in dem Sammelgefäß;
- Entnahme der Mischung aus der Vorrichtung.

Optional wird in einem weiteren Schritt die Mischung weiter aufgearbeitet. Die Aufarbeitung kann insbesondere eine Sterilfiltration, die Abtrennung des Nanocarriers und/oder der Nanoformulierung aus Mischung umfassen.

Das Verfahren wird in einem Lauf solange durchgeführt, bis sich in dem Sammelgefäß eine hinreichende Menge an Nanocarriern bzw. Nanoformulierung akkumuliert hat. Sodann wird die Mischung aus der Vorrichtung entnommen. Erforderlichenfalls wird die Mischung weiter aufgearbeitet. Im Zuge der Aufarbeitung wird insbesondere das eigentliche Zielprodukt - die Nanocarrier bzw. die Nanoformulierung - aus der Mischung abgetrennt. Die Abtrennung erfolgt nach dem Stand der Technik durch Verdampfen des flüssigen Dispersionsmediums oder durch Filtration, wie z.B. Tagential flow filtration oder durch Membrantechnologie oder durch oder durch Kombinationen daraus. Weitere geeignete Trennverfahren sind Dialyse, Sterilfiltration, Sprühtrocknung oder Lyophilisierung.

Die tertiäre Vorrichtung kann dazu verwendet werden, eine tertiäre Mischung herzustellen, wodurch ein breiteres Produktspektrum verfügbar wird. Ein zugehöriges Herstellverfahren umfasst die folgenden Schritte:
- Bereitstellen einer ersten flüssigen Phase im ersten Vorlagegefäß, wobei die erste flüssige Phase ein erstes flüssiges Dispersionsmedium enthält und wobei der pH-Wert der ersten flüssigen Phase insbesondere zwischen 6 und 8, vorzugsweise 7 beträgt;
- Bereitstellen einer zweiten flüssigen Phase im zweiten Vorlagegefäß, wobei die zweite flüssige Phase ein zweites flüssiges Dispersionsmedium und mindestens einen Präkursor eines Wirkstoffes und/oder eines Nanocarriers enthält und wobei der pH-Wert der zweiten flüssigen Phase insbesondere zwischen 3 und 5, vorzugsweise 4 beträgt;
- Bereitstellen einer dritten flüssigen Phase im dritten Vorlagegefäß, wobei die dritte flüssige Phase ein drittes flüssiges Dispersionsmedium und mindestens eine weitere Komponente umfasst, wobei die weitere Komponente ausgewählt ist aus der Gruppe bestehend aus Präkursor eines Nanocarriers, Wirkstoff, Präkursor eines Wirkstoffes;
- Antreiben der Pumpe zur Etablierung einer Flüssigkeitsströmung aus dem ersten Vorlagegefäß über den ersten Zulauf in das Wirkelement und über den Ablauf in das Sammelgefäß;
- Mischen von erster flüssiger Phase und zweiter flüssiger Phase und dritter flüssiger Phase durch Dosieren der dritten flüssigen Phase über den dritten Zulauf in das Wirkelement, wobei der Volumenstrom der dritten flüssigen Phase in dem dritten Zulauf kleiner ist als der Volumenstrom der Flüssigkeitsströmung in dem ersten Zulauf.

Die Herstellung tertiärer Systeme ist insbesondere bei der Produktion von LNP von Interesse.

Ein wesentlicher Aspekt des Verfahrens zur Herstellung tertiärer Mischungen ist, dass die erste und die zweite flüssige Phase unterschiedliche Acidität aufweisen: Die erste flüssige Phase ist eher neutral und weist eine stärkere Pufferkapazität auf als die zweite flüssige Phase, ihr pH Wert beträgt 6 bis 8, vorzugsweise 7, währenddessen die zweite flüssige Phase mit pH 3 bis 5, vorzugsweise pH 4, eher sauer ist. Da der Volumenstrom der ersten flüssigen Phase größer ist als der Volumenstrom der zweiten flüssigen Phase, findet im Wirkelement schlagartig eine Umpufferung der in der zweiten flüssigen Phase enthaltenen Präkursoren von sauer auf neutral statt. Die in-situ Umpufferung im Wirkelement ist ein besonderes Unterscheidungsmerkmal von herkömmlichen Verfahren, die in einem Mischer mit T- oder Y-förmiger Struktur ausgeführt wird: Bei diesen herkömmlichen Verfahren wird nach dem Mischen in einem separaten Schritt stabilisiert, weil die Nanocarrier in dem Milieu des Gemisches nicht lange stabil sind. Diesen Schritt spart das erfindungsgemäße Verfahren dank seiner in-situ Umpufferung ein: Durch die in-situ Umpufferung wird direkt im Wirkelement ein Milieu eingestellt, in welchem die Nanocarrier länger stabil sind. Im Zusammenspiel mit den im Wirkelement hervorgerufenen Diffusionsvorgänge kombinieren sich die ionisiert vorliegende Präkursoren zu Nanocarriern. Im Fall von LNPs sind die Lipide in dem Kontakt mit der zweiten flüssigen Phase (pH 4) kurz kationisch geladen, und binden die anionisch vorliegende RNA oder DNA aus der zweiten flüssigen Phase. Anschließend wird das Gemisch direkt durch die erste flüssige Phase erfasst und der hier vorliegende pH 7 dominiert durch die höhere Pufferkapazität in diesem Medium, wodurch die Partikel umgehend stabilisiert werden. Sofern Wirkstoffe in der ersten oder zweiten flüssigen Phase enthalten sind, werden die Nanocarrier durch diese Effekte auch mit Wirkstoff beladen. Der große Unterschied der Strömungsgeschwindigkeiten im Wirkelement bewirkt, dass die Nanocarrier bzw. die Nanoformulierung im Ablauf feinverteilt werden, sodass eine homogene Partikelgrößenverteilung im Bereich von wenigen hundert Nanometern erzielt wird.

Die erste flüssige Phase und die zweite flüssige Phase können sich nicht nur in ihrer Acidität unterscheiden, sondern auch in ihrer chemischen Natur: Die erste flüssige Phase kann eher anorganischen Charakter haben, währenddessen die zweite und/oder dritte flüssige Phase organisch ist. Bevorzugt ist sind die ersten beiden flüssigen Phasen wässrig mit unterschiedlicher Acidität und die dritte flüssige Phase organisch. Auf diese Weise ist es möglich Präkursoren und Wirkstoffe zu verwenden, die in unterschiedlichen Medien lösbar sind: So können wasserlösliche Wirkstoffe auf Carrier geladen werden, deren Präkursoren nur in organischen Lösemitteln löslich sind oder umgekehrt. Beispiele für wasserlösliche Wirkstoffe sind Peptide, Proteine, DNA und RNA wie insbesondere (m)RNA oder siRNA. Die als Carrier eingesetzten Lipide (Liposomen, LNP) oder Polymere (Partikel, Polyplexe) werden in organischen Lösungsmitteln wie beispielsweise Ethanol oder Acetonitril gelöst. In solchen Fällen handelt es sich bei dem ersten Dispersionsmedium um Wasser, währenddessen es sich bei dem zweiten Dispersionsmedium um eine organische Substanz handelt. In einem tertiären System ist meist das erste und das zweite Dispersionsmedium Wasser, währenddessen das dritte Dispersionsmedium eine organische Substanz ist.

Als organische Substanz kommt insbesondere ein einwertiger Alkohol wie beispielsweise Ethanol oder ein mehrwertiger Alkohol wie beispielsweise Glycerin in Betracht. Alternativ kann Acetonitril als organische Substanz verwendet werden oder Dimethylsulfoxid (DMSO).

Ein Beispiel für einen Präkursor eines Nanocarriers, der in organischen Lösemitteln wie Ethanol oder Glycerin lösbar ist, stellt Phosphatidylcholin (Lecithin) dar. Das Phosphatidylcholin kann aus Ei oder aus Soja gewonnen sein. Weitere Beispiele für Präkursoren von Nanocarriern, die sich in organischen Lösemitteln lösen lassen sind Cholesterol, 1,2-dioleyloxy-3-dimethylaminopropane (DODMA), Polyethylenglycol dimethacrylate (PEG-DMA) und Distearoylphosphatidylcholine (DSPC).

Zur Einstellung des pH Wertes kann die erste flüssige Phase einen Puffer enthalten. Der Puffer sorgt für ein Milieu, in dem ein Präkursor beständig ist. Der Puffer kann auch so gewählt sein, dass die Acidität des Gemisches, also nach der Vermischung der ersten und zweiten flüssigen Phase, den gewünschten Wert hat. Auf diese Weise kann der Prozess des Umpufferns entsprechend gesteuert werden Im Falle einer wässrigen ersten flüssigen Phase kann der Puffer ausgewählt sein aus der Gruppe bestehend aus Ammoniumsulfat, Acetat, Polyvinylalkohol, Phosphat, 2-(4-(2-Hydroxyethyl)-1-piperazinyl)-ethansulfonsäure.

In einer bevorzugten Weiterbildung des Verfahrens mit mindestens einer wässrigen und einer organischen Phase enthält die erste (wässrige) flüssige Phase einen Puffer und die zweite flüssige Phase (organisch) einen Wirkstoff oder einen Präkursor eines Wirkstoffes. Vorzugsweise handelt es sich bei dem Puffer um Polyvinylalkohol, und bei der organischen Substanz um Acetonitril. Als Präkursor für den Nanocarrier wird dann ein Polylactid-co-Glycolid gewählt, welches sich in Acetonitril gut lösen lässt. Es lassen sich aber auch Wirkstoffe in Acetonitril lösen, wie etwa Ritonavir. Alternativ zu Acetonitril können hier Aceton oder Dimethylsulfoxid oder Ethylacetat als organisches Lösemittel eingesetzt werden.

Es sind auch Verfahrensvarianten möglich bei denen die erste flüssige Phase (wässrig) einen Puffer enthält und bei denen die zweite flüssige Phase (alkoholisch) zwei Präkursor eines Nanocarriers enthält. So zum Beispiel kann es sich bei dem Puffer um Ammoniumsulfat handeln, währenddessen es sich bei den beiden Präkursoren des Nanocarriers um Lipoid E PC bzw. um Cholesterol HP handelt.

In tertiären Systemen mit einer ersten wässrigen Phase, einer zweiten wässrigen Phase und einer organischen Phase kann in der zweiten wässrigen Phase auch eine enthalten sein, die sowohl ein erster Präkursor eines Nanocarriers, als auch den Präkursor eines Wirkstoffes darstellt. Ein zweiter Präkursor des Nanocarriers ist dann in der dritten (organischen) Phase gelöst. Diese Konstellation wird bei der Herstellung von Nanoformulierungen angewandt, bei denen DNA oder (m)RNA auf LNP geträgert ist: Die wasserlösliche DNA bzw. (m)RNA wird in der zweiten wässrigen Phase gelöst unter sauren Bedingungen. In der organischen Phase werden Lipide gelöst. Beides wird im Wirkelement kontaktiert und in der ersten flüssigen Phase (wässrig neutral) dispergiert und zugleich umgepuffert.

Die DNA bzw. (m)RNA stellt dabei sowohl den Wirkstoff als auch ein Präkursor des Nanocarriers dar.

Unabhängig von der chemischen Natur der flüssigen Phasen kann das Verfahren kontinuierlich durchgeführt werden, wenn der Ablauf der Vorrichtung als ein Umlauf ausgeführt ist und das erste Vorlagegefäß sogleich als Sammelgefäß dient. Die Verfahrensführung erfolgt dann so, dass die erste flüssige Phase zunächst im Umlauf geführt wird, bevor die zweite flüssige Phase hinzudosiert wird. Es ist auch möglich, Produkt kontinuierlich abzuziehen, während die erste flüssige Phase und ggf. auch weitere flüssigen Phasen im Umlauf zirkuliert wird bzw. werden. Zumindest die zweite flüssige Phase wird dann ebenfalls kontinuierlich nachdosiert. In dieser Ausführungsform wird also die zweite flüssige Phase zugeführt, währenddessen zumindest die erste Phase im Umlauf geführt wird. Vorzugsweise ist der Volumenstrom der über den Umlauf zirkulierten Flüssigkeitsmenge größer als die abgezogene Produktmenge. Die nachdosierte Eduktmenge entspricht vorzugsweise der abgezogenen Produktmenge, sodass die Gesamtmenge im System konstant bleibt. Für tertiäre Systeme gilt entsprechendes.

Das durch die erfindungsgemäße Vorrichtung ermöglichte, erfindungsgemäße Verfahren führt zu einem Produkt von hoher Qualität. Es zeichnet sich insbesondere durch eine besonders homogene Partikelgrößenverteilung und ggf. auch durch eine gleichmäßige Wirkstoffkonzentration aus. Gegenstand der Erfindung ist mithin auch ein Produkt, welches durch ein erfindungsgemäßes Verfahren erhältlich ist. Bei dem Produkt des Verfahrens handelt es sich um einen Nanocarrier oder um eine Nanoformulierung.

Das erhaltene Produkt ist nanoskalig. Es weist eine mittlere Partikelgröße kleiner als 300 nm auf, vorzugsweise kleiner als 200 nm, besonders bevorzugt zwischen 40 nm und 140 nm. Mit einem bestimmten Typ Nanocarrier geht stets eine bestimmte Größenordnung der Partikelgröße einher. Die Partikelgrößenverteilung wird gemessen nach dem Prinzip der dynamischen Lichtstreuung mit dem Messgerät Zetasizer der Fa. Malvern Panalytical Ltd, GB.

Das erhaltene Produkt zeichnet sich durch einen Polydispersitätsindex zwischen 0.08 und 0.2 auf. Dies spricht für eine gleichbleibende Produktqualität. Der Polydispersitätsindex (PDI) wird gemessen nach dem Prinzip der dynamischen Lichtstreuung mit dem Messgerät Zetasizer der Fa. Malvern Panalytical Ltd, GB. Besonders bevorzugt ist ein PDI von 0.1 bis 0.3.

Das Verfahren ermöglicht die Herstellung von Nanoformulierungen, die eine hohe Wirkstoffkonzentration aufweisen. Vorzugsweise beträgt die massenbezogene Wirkstoffkonzentration der erfindungsgemäß erhältlichen Nanoformulierung von 1 ppm bis 50%. Gemessen wird dieser Wert mittels folgt: HPLC-UV, Beispielsweise mit einer AGILENT 1260 series Apparatur. Die große Spanne der möglichen Wirkstoffkonzentration ist der Tatsache geschuldet, dass manche Wirkstoffe wie z.B. mRNA in sehr geringen Dosen verabreicht werden.

Ein Produkt mit hoher Wirkstoffkonzentration kann auch deshalb hergestellt werden, weil das Verfahren eine hohe Beladungseffizienz erreicht. Dies bedeutet, dass der vorgelegte Wirkstoff zu einem hohen Anteil auf die Nanocarrier geladen wird und nicht verloren geht

Die erfindungsgemäße Vorrichtung soll nun anhand von Gestaltungsbeispielen erläutert werden. Hierfür zeigen:
- **Fig. 1:**: erste Variante einer erfindungsgemäßen Vorrichtung
- **Fig. 2:**: zweite Variante einer erfindungsgemäßen Vorrichtung;
- **Fig. 2x:**: zweite Variante einer erfindungsgemäßen Vorrichtung, Detail Wirkelement und Zuläufe;
- **Fig. 2z:**: zweite Variante einer erfindungsgemäßen Vorrichtung,Schnitt durch Zuläufe
- **Fig. 3:**: dritte Variante einer erfindungsgemäßen Vorrichtung;
- **Fig. 4a:**: Querschnitt Vertikalabschnitt binäre Vorrichtung, schematisch;
- **Fig. 4b:**: Querschnitt Vertikalabschnitt 1. Variante tertiäre Vorrichtung, schematisch;
- **Fig. 4c:**: Querschnitt Vertikalabschnitt 2. Variante tertiäre Vorrichtung, schematisch;
- **Fig. 4d:**: Querschnitt Vertikalabschnitt 3. Variante tertiäre Vorrichtung, schematisch;
- **Fig. 5a:**: Wirkelement in erster Axialposition;
- **Fig. 5b:**: Wirkelement in zweiter Axialposition;

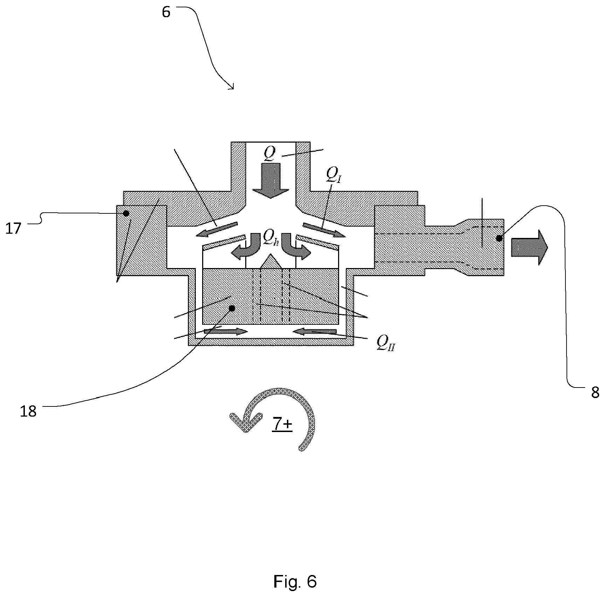

- **Fig. 6:**: Prinzipskizze levitronische Pumpe;
- **Fig. 7a:**: Strömungssimulation, erstes Schnittbild;
- **Fig. 7b:**: Strömungssimulation, zweites Schnittbild;
- **Fig. 7c:**: Strömungssimulation, drittes Schnittbild;
- **Fig. 8:**: Flow-Vektor-Bild.

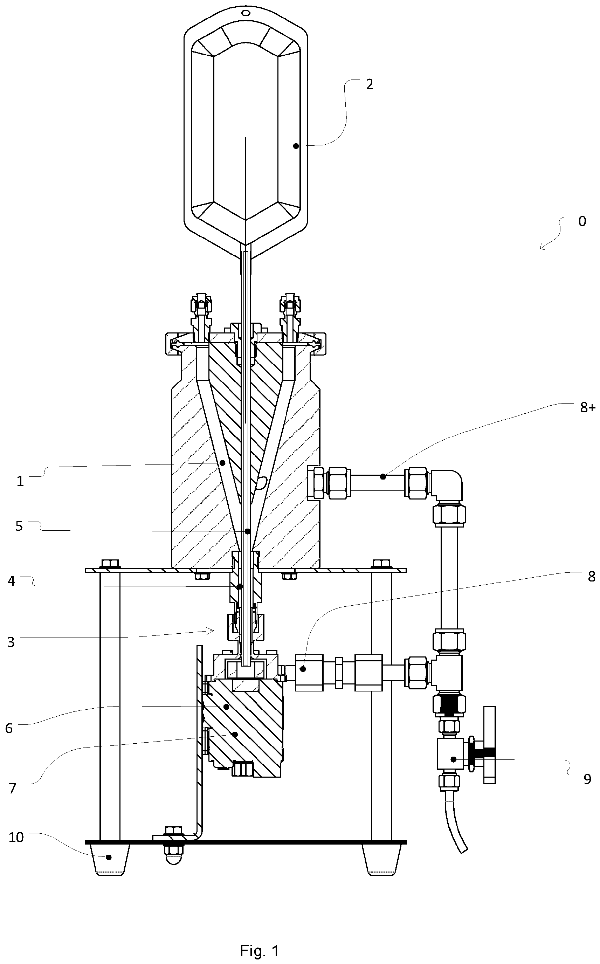

Figur 1 zeigt eine Übersicht einer ersten erfindungsgemäßen Vorrichtung 0. Sie umfasst einen erstes Vorlagegefäß 1 in Gestalt eines trichterförmigen Tanks und ein zweites Vorlagegefäß 2 in Gestalt von einem Kunststoffbeutel. Die beiden Vorlagegefäße 1, 2 dienen zur Aufnahme einer erster bzw. zweiten flüssigen Phase. Das Volumen des ersten Vorlagegefäßes 1 ist deutlich größer als das Volumen des zweiten Vorlagegefäßes 2. Die flüssigen Phasen sind jeweils Dispersionen enthaltend ein flüssiges Dispersionsmittel und Präkursoren von Nanocarrier und/oder Wirkstoffe.

Unterhalb der Vorlagegefäße 1, 2 (vertikal in Richtung der Erdbeschleunigung) ist ein Wirkelement 3 angeordnet. Das Wirkelement 3 dient zum Mischen der beiden flüssigen Phasen. Eine erste Zuleitung 4 und eine zweite Zuleitung 5 verbinden das erste Vorlagegefäß 1 bzw. das zweite Vorlagegefäß mit dem Wirkelement 3. Die flüssigen Phasen können aus ihren jeweiligen Vorlagegefäßen 1,2 durch die jeweilige Zuleitung 4,5 zu dem Wirkelement 3 fließen. Beide Zuleitungen 4, 5 verlaufen vertikal.

Unterhalb des Wirkelements 3 (vertikal in Richtung der Erdbeschleunigung) ist eine Pumpe 6 angeordnet. Die Pumpe 6 dient zum Bewegen des von dem Wirkelement 3 bereiteten Gemisches. Wirkelement 3 und Pumpe 6 gehen direkt ineinander über. Die Pumpe 6 kann von einem integrierten Antrieb 7 drehend angetrieben werden.
Stromabwärts der Pumpe 6 ist ein Ablauf 8 vorgesehen, der ein Stück weit horizontal verläuft und sodann zum ersten Vorlagegefäß 1 zurückführt. Der Ablauf 8 ist folglich als Umlauf 8+ ausgeführt. Der Ablauf 8 ist mit einer Entnahmearmatur 9 versehen.

Die gesamte Vorrichtung 0 ist in einem Gestell 10 untergebracht. Nicht zu erkennen ist eine Steuerung und eine Dosiervorrichtung zum Dosieren der zweiten flüssigen Phase. Die Steuerung steuert den Volumenstrom der Pumpe 6 und den Volumenstrom der Dosiervorrichtung. Der Volumenstrom der Pumpe 6 entspricht dem Volumenstrom des Gemisches aus erster und zweiter flüssiger Phase durch den als Umlauf ausgeführten Ablauf 8. Innerhalb des Gemisches ist der Anteil der ersten flüssigen Phase deutlich größer als der Anteil der zweiten flüssigen Phase. Somit entspricht der Volumenstrom der Pumpe (unter Vernachlässigung der zweiten flüssigen Phase) dem Volumenstrom der ersten flüssigen Phase. Die Pumpe dient somit quasi als eine Art Dosiervorrichtung für die erste flüssige Phase.

Im Betrieb wird mittels der Pumpe 6 zunächst ein Umlauf der ersten flüssigen Phase aus dem ersten Vorlagegefäß 1 über die erste Zuleitung 4 in das Wirkelement 3 und über den Ablauf 8 / Umlauf 8+ zurück in das erste Vorlagegefäß 1 etabliert. Sodann wird eine geringe Menge der zweiten flüssigen Phase aus dem zweiten Vorlagegefäß 2 über die zweite Zuleitung 5 in das Wirkelement 3 dosiert Die beiden Phasen werden in dem Wirkelement 3 vermischt, sodass ein Gemisch entsteht. Das Gemisch enthält Dispersionsmittel und darin gelöst Nanocarrier oder Nanoformulierung, welches durch Kontakt der Präkursoren im Wirkelement 3 entsteht.

Der Umlauf wird solange gefahren bis sich eine gewünschte Menge an Produkt im Gemisch akkumuliert hat. Sodann wird das Gemisch über die Entnahmearmatur 9 entnommen und das Produkt aus dem Gemisch abgetrennt. Dies geschieht außerhalb der Vorrichtung 0 mit bekannten Trennapparaten. Es ist auch möglich das Gemisch kontinuierlich aus dem Umlauf abzuziehen.

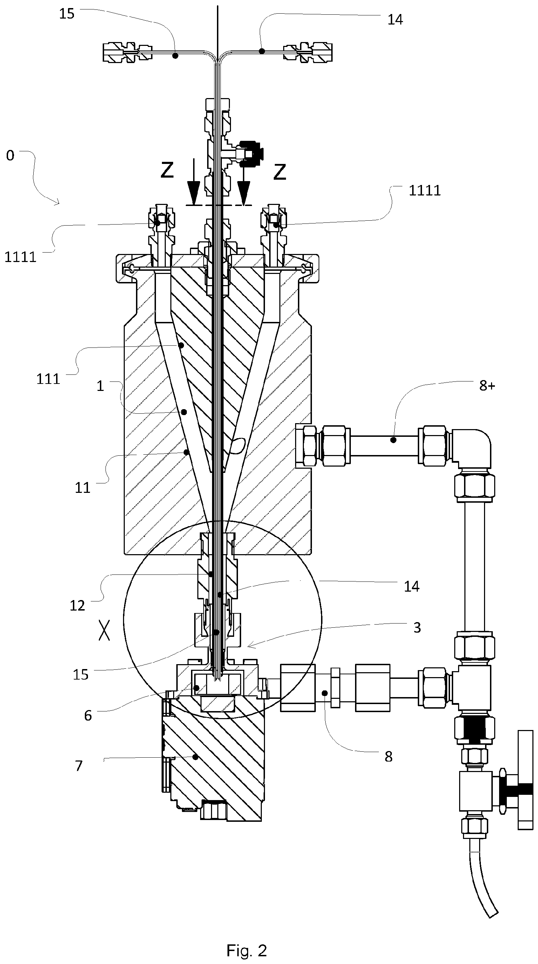

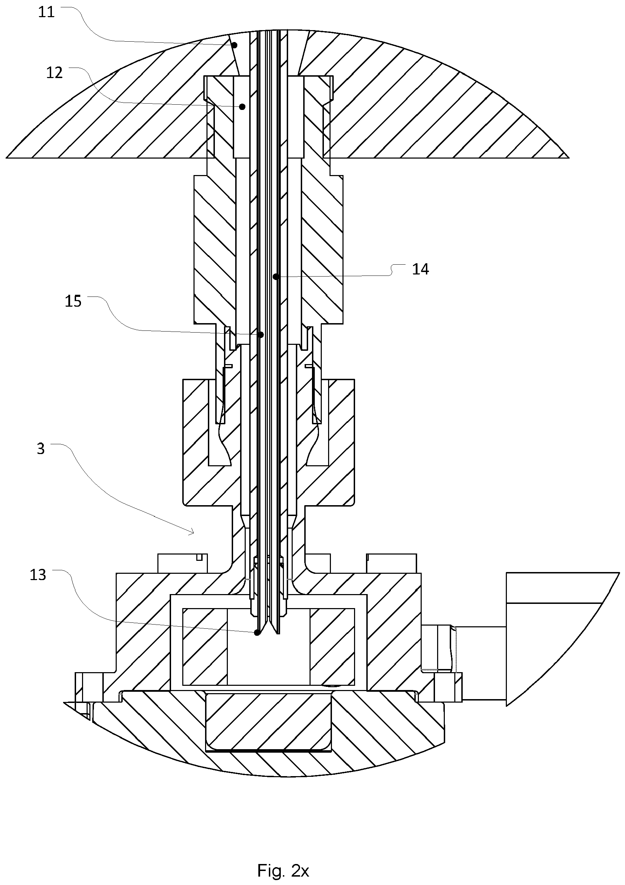

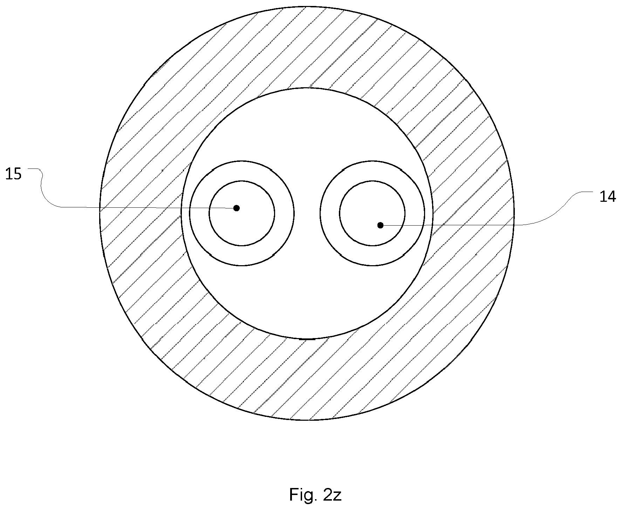

Figur 2 zeigt eine tertiäre Ausführungsform der Vorrichtung 0. Diese ermöglicht die Herstellung von Gemischen aus drei flüssigen Phasen.

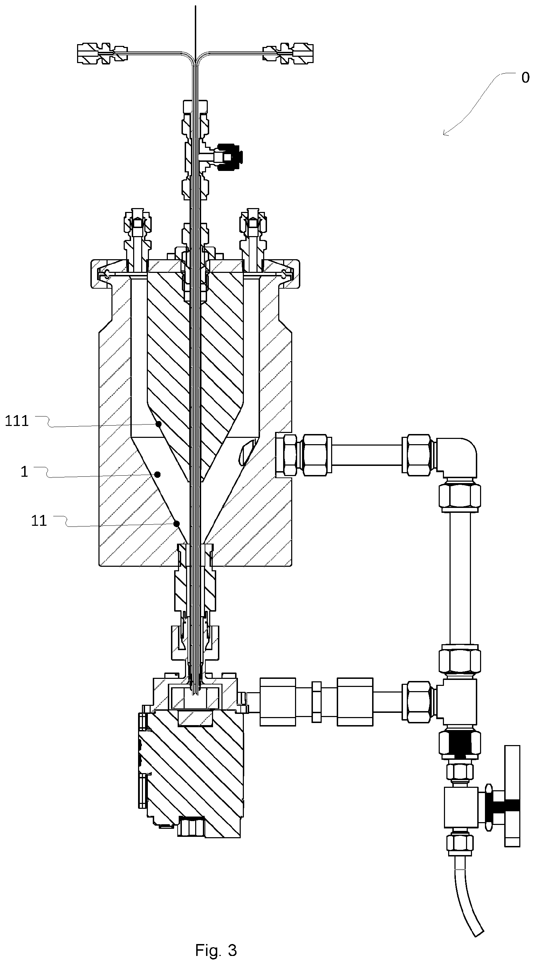

Die erste flüssige Phase wird dem ersten Vorlagegefäß 1 gefüllt, das wieder trichterförmig ausgeführt ist, wie ein Silo. Dementsprechend weißt das erste Vorlagegefäß innen einen Konus 11 auf. Der Winkel des Konus 11 kann in Abhängigkeit von der Viskosität der ersten flüssigen Phase unterschiedlich gewählt sein. In Figur 2 beträgt der Winkel 29°, in Figur 3 beträgt der Winkel 55°, es sind aber auch Winkel bis 86° denkbar. Das Volumen des ersten Vorlagegefäßes 1 ist durch einen eingesetzten Dorn 111 begrenzt. Der Dorn 111 ist ebenfalls konisch und weist denselben Winkel auf wie Konus 11. Der Dorn 111 kann axial versetzt eingebaut werden, um das Volumen des ersten Vorlagegefäßes 1 zu variieren. Dies ist insbesondere dann erforderlich, wenn die Vorrichtung 0 für die Herstellung unterschiedlicher Nanoformulierungen verwendet wird, die sich durch ihre Wirkstoffkonzentration unterscheiden. Im Umlaufbetrieb lässt sich außerdem der hold up des ersten Vorlagegefäßes durch den Dorn 111 einstellen. Darüber hinaus ist das erste Vorlagegefäß 1 mit zwei Vorlagearmaturen 1111 versehen, durch welche erste flüssige Phase in das erste Vorlagegefäß gefüllt bzw. nachgefüllt werden kann. Die Vorrichtung 0 erlaubt einen kontinuierlichen Betrieb, in dem die erste flüssige Phase dauerhaft über die Vorlagearmaturen 1111 zugeführt wird. Am Fuße des Konus 11 geht das erste Vorlagegefäß in eine erste Rohrleitung 12 über. Das ist gut in der Vergrößerung der Figur 2x erkennbar.

Die Vorlagegefäße für die zweite und dritte flüssige Phase sind in Figur 2 nicht dargestellt. Zu erkennen sind aber eine zweite Rohrleitung 14 und eine dritte Rohrleitung 15, die als Zulauf für zweite flüssige Phase (über Rohrleiung 14) bzw. dritte flüssige Phase (über Rohrleitung 15) in das Wirkelement 3 dienen.

Entsprechend durchmessen die beiden Rohrleitungen 14 und 15 die gesamte Vorrichtung bis zum Wirkelement 3. Zwischen Fuß des Konus 11 und dem Wirkelement 3 verlaufen die zweite Rohrleitung 14 und die dritte Rohrleitung 15 vertikal und innerhalb der ersten Rohrleitung 12. Dieser Bereich wird als Vertikalabschnitt bezeichnet, weil alle drei Rohrleitungen 12, 14 und 15 in Richtung der Erdbeschleunigung verlaufen. Die Strömungsvektoren der drei flüssigen Phasen unmittelbar vor ihrem Kontakt im Wirkelement ist demnach parallel zum Gravitationsvektor.

Im Übrigen verlaufen die Rohrleitungen 14 und 15 auch oberhalb des Fußes des Konus 11 parallel und vertikal, das ist aber nicht relevant. Figur 2z zeigt einen Querschnitt durch die zweite und dritte Rohrleitung 14, 15 oberhalb des Vertikalabschnitts. Das in Figur 2z gezeigte schraffierte Bauteil ist eine Armierung.

An ihrer jeweiligen Mündung im Wirkelement 3 sind die beiden Rohrleitungen 14 und 15 jeweils mit einem V-förmigen Schliff 13 versehen. Dieser lässt sich in Figur 2x besser erkennen. Der Winkel des Schliffes 13 kann 15° oder 30° oder 60° betragen.

Es ist denkbar durch die zweite Rohrleitung 14 und die dritte Rohrleitung 15 jeweils zweite flüssige Phase zu leiten. Zweite und dritte Rohrleitung sind dann jeweils eine Ader einer zweiadrigen zweiten Zuleitung einer binären Vorrichtung zur Herstellung einer zweiphasigen Mischung.

Figur 3 zeigt eine dritte erfindungsgemäße Ausführungsform der Vorrichtung 0. Sie entspricht im Wesentlichen der in Figur 2 gezeigten zweiten Variante. Unterschiedlich ist jedoch das Volumen des ersten Vorlagegefäßes 1, welches bei der in Figur 3 gezeigten Variante größer ist als bei Figur 2.

Das größere Volumen ist dadurch eingestellt worden, dass der Dorn 111 weiter oben positioniert wurde. Dadurch wird der Raum zwischen dem Konus 11 des ersten Vorlagegefäßes und des korrespondierenden Konus des Dorns 111 größer. Des Weiteren ist der Konuswinkel bei der in Figur 3 gezeigten Variante größer; er beträgt hier 55° statt lediglich 29° in Figur 2.

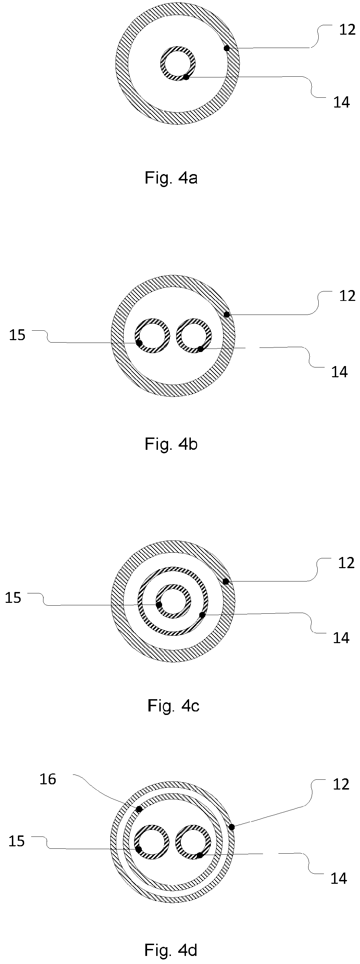

Die Figuren 4a bis 4b zeigen schematisch mögliche Ausführungsformen des Vertikalabschnitts im Querschnitt, also in Blickrichtung der Erdbeschleunigung. Alle Rohrleitungen 12, 14, 15 sind hier kreisrund dargestellt, sie können aber auch eckig sein.

Der in Figur 4a gezeigte Vertikalabschnitt gehört zu einer binären Vorrichtung mit einer ersten Rohrleitung 12 für die erste flüssige Phase und eine zweite Rohrleitung 14 für die zweite flüssige Phase. Die zweite Rohrleitung 14 verläuft innerhalb der ersten Rohrleitung 12. Beide Rohrleitungen sind koaxial angeordnet, das bedeutet, der geometrische Mittelpunkt ihrer Querschnitte ist identisch. Die erste flüssige Phase fließt innerhalb der ersten Rohrleitung 12 und außerhalb der zweiten Rohrleitung 14. Die zweite flüssige Phase fließt innerhalb der zweiten Rohrleitung 14. Die in Figur 4a gezeigte Konstellation wird auch "Einfachkapillare" genannt.

Der in Figur 4b gezeigte Vertikalabschnitt genannt "Doppelkapillare" gehört zu einer tertiären Vorrichtung mit einer ersten Rohrleitung 12 für die erste flüssige Phase, einer zweite Rohrleitung 14 für die zweite flüssige Phase und einer dritte Rohrleitung 15 für die dritte flüssige Phase. Die zweite Rohrleitung 14 verläuft innerhalb der ersten Rohrleitung 12 und die dritte Rohrleitung 15 verläuft ebenfalls innerhalb der ersten Rohrleitung 12. Die zweite Rohrleitung 14 und die dritte Rohrleitung 15 verlaufen parallel. Die erste flüssige Phase fließt innerhalb der ersten Rohrleitung 12 und außerhalb der zweiten Rohrleitung 14 und der dritten Rohrleitung 15. Die zweite flüssige Phase fließt innerhalb der zweiten Rohrleitung 14. Die dritte flüssige Phase fließt innerhalb der dritten Rohrleitung 15. Es ist auch möglich, sowohl durch die zweite Rohrleitung 14 und die dritte Rohrleitung 15 dieselbe zweite flüssige Phase zu leiten. Dann wird die Vorrichtung mit der Doppelkapillare lediglich binär genutzt.

Der in Figur 4c gezeigte Vertikalabschnitt gehört zu einer tertiären Vorrichtung mit einer ersten Rohrleitung 12 für die erste flüssige Phase, einer zweite Rohrleitung 14 für die zweite flüssige Phase und einer dritte Rohrleitung 15 für die dritte flüssige Phase. Die zweite Rohrleitung 14 verläuft innerhalb der ersten Rohrleitung 12 und die dritte Rohrleitung 15 verläuft ebenfalls innerhalb der ersten Rohrleitung 12 und zusätzlich auch innerhalb der zweiten Rohrleitung 14. Alle Rohrleitungen 12, 14, 15 sind koaxial zueinander angeordnet. Die erste flüssige Phase fließt innerhalb der ersten Rohrleitung 12 und außerhalb der zweiten Rohrleitung 14. Die zweite flüssige Phase fließt innerhalb der zweiten Rohrleitung 14 und außerhalb der dritten Rohrleitung 15. Die dritte flüssige Phase fließt innerhalb der dritten Rohrleitung 15.

Der in Figur 4d gezeigte Vertikalabschnitt gehört zu einer tertiären Vorrichtung mit einer ersten Rohrleitung 12 für die erste flüssige Phase, einer zweite Rohrleitung 14 für die zweite flüssige Phase und einer dritte Rohrleitung 15 für die dritte flüssige Phase. Die zweite Rohrleitung 14 verläuft innerhalb der ersten Rohrleitung 12 und die dritte Rohrleitung 15 verläuft ebenfalls innerhalb der ersten Rohrleitung 12. Die zweite Rohrleitung 14 und die dritte Rohrleitung 15 verlaufen parallel. Die erste Rohrleitung 12 weist hier eine Innenwandung 16 auf. Der Strömungsquerschnitt der ersten Rohrleitung ist daher ringkreisförmig. Die erste flüssige Phase fließt außerhalb der Innenwandung 16 der ersten Rohrleitung 12 und innerhalb der Außenwandung der ersten Rohrleitung 12, also der schraffierten Schnittfläche mit der Position 12. Zwischen der Innenwandung 16 und der Wandung der zweiten bzw. dritten Rohrleitung 14,15 findet kein Materialfluss statt. Die zweite flüssige Phase fließt innerhalb der zweiten Rohrleitung 14. Die dritte flüssige Phase fließt innerhalb der dritten Rohrleitung 15.

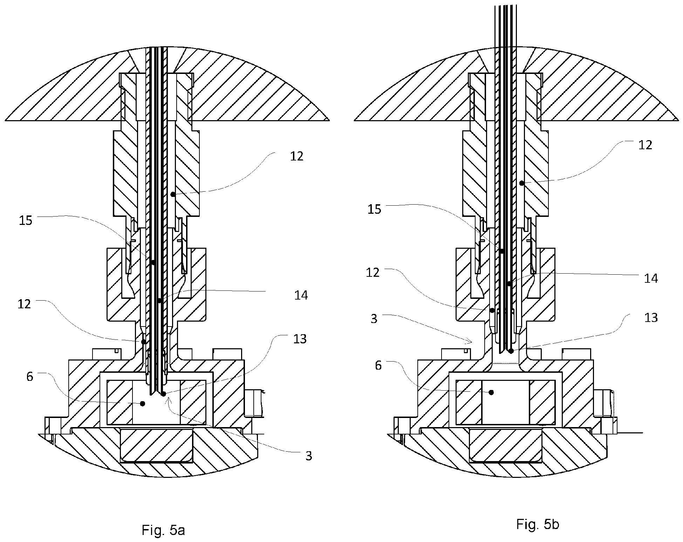

In den Figuren 5a und 5b ist dargestellt, wie sich bei den in den Figuren 2 und 3 dargestellten teritären Vorrichtungen die zweite Rohrleitung 14 zusammen mit der dritten Rohrleitung 15 gegenüber der ersten Rohrleitung 12 axial verschieben lässt: In Figur 5b ist der vertikale Abstand der Mündung mit dem Schliff 13 zur Pumpe 6 größer als bei Figur 5a, wo die Mündung in die Pumpe 6 hineinragt. Dies wird mit einem hier nicht dargestellten Linearabtrieb bewerkstelligt. Die erste Rohrleitung 12 bleibt dabei unbewegt. Die Parallelität und vertrikale Ausrichtung aller Rohrleitungen 12, 14, 15 bleibt stets erhalten.

Es ist auch denkbar, die zweite Rohrleitung 14 und die dritte Rohrleitung 15 getrennt voneinander axialverstellbar auszuführen. Das ist bei den hier dargestellten Ausführungsformen nicht möglich; die die zweite Rohrleitung 14 und die dritte Rohrleitung 15 können nur als Paket verstellt werden. Alternativ zur Verstellung der zweiten und/oder dritten Rohrleitung kann auch die erste Rohrleitung 12 axial verstellbar ausgeführt werden. Es kommt nämlich im maßgeblich auf die relative axiale Ausrichtung der Rohrleitungen zueinander an, nicht auf die absolute Position. Bei den hier gezeigten Ausführungsformen ist die erste Rohrleitung 12 aber ortsfest.

Durch die Variation der axialen Ausrichtung der beiden Rohrleitungen 12, 14 zueinander werden die Strömungsverhältnisse im Wirkelement 3 variiert und damit die Bildung der Nanocarrier bzw. der Nanoformulierung optimiert.

Figur 5 ist eine Prinzipskizze der Pumpe 6. Die Pumpe 6 ist als Kreiselpumpe ausgeführt. Sie umfasst ein unbewegliches Gehäuse 17, in dem ein Läufer 18 drehbar gelagert ist. Der Läufer 18 kann auch als Pumpenrad bezeichnet werden. Der Antrieb und die Lagerung des Läufers 18 erfolgt über ein Drehfeld 7*. Die Drehachse des Läufers 18 ist vertikal ausgerichtet. Vorzugsweise verläuft die Drehachse koaxial zu der Achse der ersten Rohrleitung 12. Dies ist bei den in den Figuren 1, 2 und 3 gezeigten Varianten der Fall.

Eine Besonderheit der Pumpe 6 ist, dass diese levitronisch ausgeführt ist. Dies bedeutet, dass der Läufer 18 nicht über eine starre Welle an dem Antrieb 7 gekuppelt ist, sondern magnetisch über das Drehfeld 7*. Das umlaufende Drehfeld 7* schleppt den Läufer 18 mit. Auf diese Weise lässt sich der Läufer 18 berührungsfrei lagern und in eine Drehbewegung um seine vertikale Drehachse versetzen.

Im Betrieb läuft von oben das Gemisch in das Gehäuse 17 der Pumpe 6 ein. Von dem drehenden Läufer 18 wird das Gemisch in einer Horizontalebene radial nach außen beschleunigt und verlässt das Gehäuse 16 wieder über den Ablauf 8. Ein Teil des Gemisches umströmt den Läufer 18 auf dessen Unterseite. Dies ist dank der magnetischen Lagerung und Kraftübertragung möglich.

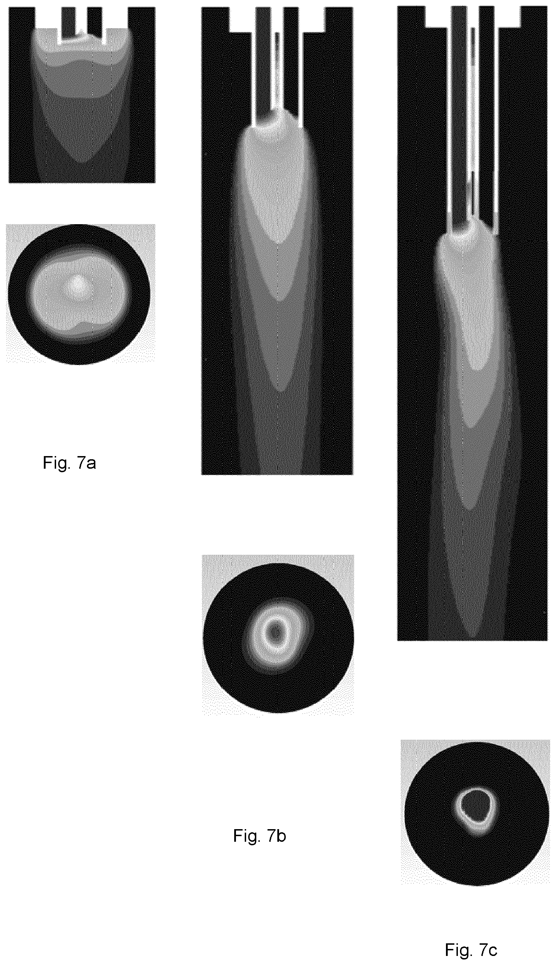

Die Figuren 7a, 7b und 7c zeigen eine Strömungssimulation durch ein Wirkelement 3 der in Figur 3 dargestellten Vorrichtung: Die Darstellung zeigt die örtliche Konzentration von Ethanol, das über die linke Kapillare zugeführt wird. In Abhängigkeit von der Kapillarlänge außerhalb der Lanze variiert die "Ethanolkonzentration" in allen drei Raumrichtungen. Die drei Schnittbilder zeigen den Querschnitt jeweils ca. 2 mm nach Ende der Kapillaren. Das erste Schnittbild in Figur 7a zeigt, dass das Ethanol bereits nach ca. 2 mm Wegstrecke nur noch 1 Mol-Prozent Konzentrationsunterschied in der Hauptströmung beobachtbar ist. Das in Figur 7b gezeigte zweite Schnittbild (Kapillarlänge 10 mm) zeigt nach 2 mm eine in die Länge gezogene Kernströmung, bei der der Konzentrationsunterschied ca. 6 mol-Prozent beträgt. Das in Figur 7c gezeigte dritte Schnittbild (Kapillarlänge 20 mm) zeigt nach 2 mm eine in die Länge gezogene Kernströmung, bei der der Konzentrationsunterschied ca. 10 mol-Prozent beträgt.

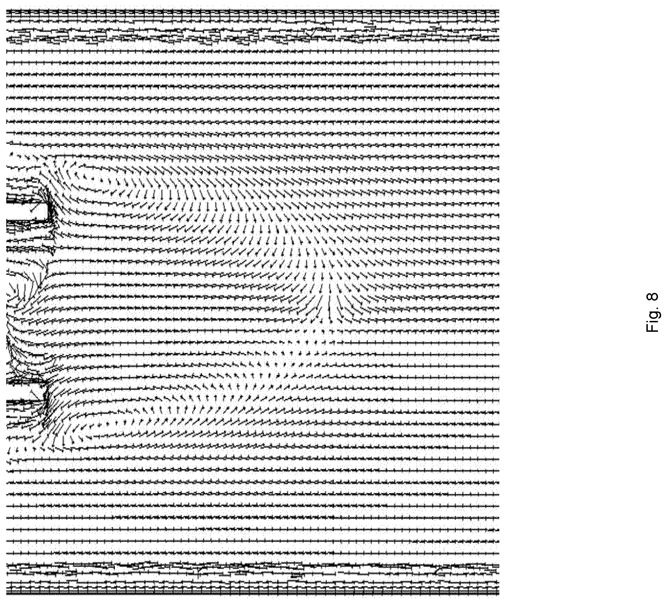

Figur 8 zeigt ein Flow-Vektor-Bild einer Vorrichtung mit zwei 3 mm-Kapillaren im Bereich des Wirkelements. Die Geschwindigkeitsvektoren der Flüssigkeitsströmung sind darin als kleine Pfeile dargestellt. Der Fachmann erkennt, dass die Strömungspfade der ersten flüssigen Phase (außen) weitestgehend parallel/vertikal bleiben. Nur im Bereich der Zuleitung der zweiten bzw. dritten flüssigen Phase (mittig) über die beiden Kapillaren kommt es zu einer homogenen Abweichung der Strömungsrichtung, die aber nicht als turbulent bezeichnet werden kann.

### Beispiele:

Das erfindungsgemäße Verfahren wird nun anhand von experimentellen Beispielen näher erläutert. Tabelle 0 enthält eine Übersicht der Beispiele.

Alle Experimente wurden mit einer Vorrichtung entsprechend der Figuren 2 bzw. 3 ausgeführt. Das Innenvolumen der Vorlage 1 wurde durch Verstellung des Dorns 111 variiert. Die Vorrichtung wies eine levitronische Pumpe der Fa. Levitronix auf. Es wurden zusätzlich Dosiervorrichtungen zum Dosieren der ersten und zweiten flüssigen Phase eingesetzt. Diese sind den Zeichnungen nicht dargestellt.

Mit dem Messgerät Zetasizer der Fa. Malvern Panalytical Ltd, GB wurden jeweils Polydispersität (PDI) und mittlere Partikelgröße (Zav) bestimmt. Der Winkel für Backsatter wurde auf 173° eingestellt.

**Tabelle 0: Übersicht der Beispiele**

| **Beispiel** | | **Erste flüssige Phase** | | | **Zweite flüssige Phase** | | | | **Dritte flüssige Phase** | |
|---|---|---|---|---|---|---|---|---|---|---|
| Gruppe | Nr | Dispersionsmedium | Puffer | Wirkstoff | Dispersionsmedium | Puffer | Präkursor Nanocarrier | Wirkstoff | Dispersionsmedium | Präkursor Nanocarrier / Wirkstoff |
| 1 | I | Wasser | Ammoniumsulfat | Entfällt | Ethanol | Entfällt | EPC Chol | Entfällt | Entfällt | Entfällt |
| 1 | II | Wasser | Ammoniumsulfat | Entfällt | Ethanol | Entfällt | EPC Chol | Entfällt | Entfällt | Entfällt |
| 1 | III | Wasser | Ammoniumsulfat | Entfällt | Ethanol | Entfällt | EPC Chol | Entfällt | Entfällt | Entfällt |
| 2 | IV | Wasser | Entfällt | Entfällt | Ethanol | Entfällt | NAT 8539 | Entfällt | Entfällt | Entfällt |
| 2 | V | Wasser | Entfällt | Entfällt | Ethanol | Entfällt | NAT 8539 | Entfällt | Entfällt | Entfällt |
| 3 | VI | Wasser | PVA | Entfällt | Acetonitril | Entfällt | PLGA | Ritonavir | Entfällt | Entfällt |
| 4 | VII | Wasser | Phosphat | Entfällt | Wasser | Acetat | Poly A | Poly A | Ethanol | DODMA, DSPC, Cholesterol, PEG-DMG |
| 4 | VIII | Wasser | Phosphat | Entfällt | Wasser | Acetat | Poly A | Poly A | Ethanol | DODMA, DSPC, Cholesterol, PEG-DMG |
| 5 | IX | Wasser | Entfällt | Entfällt | Wasser | Acetat | PEI | Entfällt | Wasser | Poly A |
| 5 | X | Wasser | Entfällt | Poly A | Wasser | Acetat | PEI | Entfällt | Entfällt | Entfällt |

### Legende für Tabelle 0:

PVA = Polyvinylalkohol
EPC Chol = Lipoid E PC und Cholesterol HP
NAT 8539 = Soja Phosphatidylcholin
PLGA = Poly(D,L-lactide-co-glycolide)
Poly A = Poly- Adenosinmonophosphat
DODMA = 1,2-dioleyloxy-3-dimethylaminopropane
DSPC = Distearoylphosphatidylcholine
PEG-DMG = Polyethylenglycol dimethacrylate
PEI = Polyethylenimin

### Beispielgruppe 1: Herstellung von Liposomen als Nanocarrier für Pharma-Anwendungen

Wie Beispiel I bis III zeigen, lassen sich mit Hilfe der levitronischen Pumpe Liposomen mit einem robusten Prozess über einen weiten Konzentrationsbereich an Lipiden generieren. Als Beispielrezeptur wurden hier Lipoid E PC und Cholesterol HP im Verhältnis (55 mol%:45 mol%) in variierender Konzentration eingesetzt.

### Beispiel I:

Im Beispiel I wurden 700 ml eines 250 mM Ammoniumsulfatpuffer als erste flüssige Phase vorgelegt und 77,5 ml zweite flüssige Phase mit 200 mg/ml EPC-Chol in Ethanol hinzudosiert. Dabei betrug der stationäre Volumenfluss der ersten flüssigen Phase rund 8 Liter pro Minute in den dann 10 ml pro Minute zweite flüssige Phase über einen Zeitraum von rund 8 Minuten zugegeben wurde. Die Hinzudosierung erfolgt mittels eines formstabilen Schlauchs oder einer Kapillare oberhalb der levitronischen Pumpe. Dabei konnte eine Liposomendispersion mit einer mittleren Teilchengröße von ca. 150 nm hergestellt werden. Das Ergebnis ließ sich auch bei halbierter Zudosierungsrate und auf 183.2 ml reduzierten Chargengröße reproduzieren. Dies belegt, dass die Vorrichtung ein gutes Upscaling ermöglicht. Ergebnisse sind in Tabelle 1 niedergelegt.

### Beispiel II:

Im Beispiel II ist bei gleichbleibendem Lipidverhältnis EPC-Chol (55 mol%:45 mol%) die Lipidkonzentration in der organischen Phase und somit auch im Produkt verdoppelt. Die resultierenden Partikel sind bei vergleichbaren Prozessparametern wie im Beispiel I größer und weisen eine höhere Polydispersität auf. Des Weiteren wurde in diesem Beispiel der Einfluss der Pumpengeschwindigkeit/stationärer Volumenfluss der ersten flüssigen Phase untersucht. Es konnte gezeigt werden, dass für diese Rezeptur höhere Pumpraten zu besseren Ergebnissen führen. Ergebnisse sind in Tabelle 2 niedergelegt.

### Beispiel III:

Im Beispiel III ist bei gleichbleibendem Lipidverhältnis EPC-Chol (55 mol%:45 mol%) die Lipidkonzentration in der organischen Phase und somit auch im Produkt reduziert. Der Lipidgehalt der organischen Phase beträgt 15.2 mg/ml und außerdem wird das Verhältnis von wässriger zu organischer Phase variiert, sowie erneut der Einfluss der Pumpengeschwindigkeit stationärer Volumenfluss der ersten Phase untersucht. Außerdem wurde mit einer zweiten Kapillare mit reduziertem Innendurchmesser gearbeitet. Die Ergebnisse spiegeln einen robusten Prozess mit Ergebnissen zwischen 79.85 nm und 108.4 nm wieder. Die geringste Partikelgröße wurde mit einer feinen Kapillare zur Eindosierung der organischen Phase erzielt. Ergebnisse sind in Tabelle 3 niedergelegt.

### Beispielgruppe 2: Liposomale Trägersysteme für kosmetische Anwendungen

Phospholipide sind wichtige Bestandteile der Zellmembranen und somit Körperverwand. Sie können als Bausteine von Liposomen als Träger für schwerlösliche kosmetisch wirksame Stoffe wie bspw. Vitamin A & E oder Coenzym Q10 benutzt werden und verbessern die Hautpenetration. Diese Eigenschaften der Liposomen finden neben der Formulierung von Arzneimitteln zunehmend Anwendung in der Kosmetik. Die phospholipide beinhaltenden Liposomen besitzen auch eigene biologische Wirkungen, was für die normale Funktion der Haut eine wichtige Bedeutung hat. Aus diesem Grund finden "leere" Liposomen ohne Inhaltsstoffe ihren Platz als Feuchtigkeitsspender in der Kosmetik.

Wie Beispiel IV und V zeigen, lassen sich mit Hilfe der levitronischen Pumpe Phospholipide einer kosmetischen Standardformulierung in Liposomen verarbeiten. Als Beispielrezeptur wurde hier NAT 8539 (aufgereinigtes Soja Phosphatidylcholin in Ethanol) in variierender Konzentration eingesetzt.

### Beispiel IV:

Im Beispiel IV wurden zur erfolgreichen Herstellung von Liposomen 583 g Wasser in als erste flüssige Phase vorgelegt und 212 g zweite flüssige Phase bestehend aus 107 g NAT 8539 (ethanolische Lösung) und 105 g Ethanol hinzudosiert. Die Hinzudosierung erfolgt mittels einer Kapillare oberhalb der levitronischen Pumpe. In untenstehender Tabelle sind die Ergebnisse verschiedener Anordnungen dargestellt. Eine höhere Rate der Hinzudosierung resultiert tendenziell in kleineren Partikeln. Hohe Flussraten (rpm) führen zu geringerer Partikelgröße und engerer Partikelgrößenverteilung. Der Einfluss des Abstands zwischen Kapillare und Pumpenkopf ist eine zu vernachlässigende Größe. Die Ergebnisse sind in Tabelle 4 niedergelegt.

### Beispiel V:

Im Beispiel V wurden zur erfolgreichen Herstellung von Liposomen 583 g Wasser als erste flüssige Phase vorgelegt und 212 g zweite flüssige Phase bestehend aus 53.5 g NAT 8539 (ethanolische Lösung) und 158.5 g Ethanol hinzudosiert. Die Lipidkonzentration in der organischen Phase und somit auch im Produkt wurde somit halbiert. Die Hinzudosierung erfolgt mittels einer Kapillare oberhalb der levitronischen Pumpe. Die resultierenden Partikel sind < 200nm und weisen einen PDI unterhalb von 0.2 auf. Die Ergebnisse sind in Tabelle 5 niedergelegt.

### Beispielgruppe 3: Bioabbaubare Nanopartikel als Arzneiträgersysteme für pharmazeutische Anwendungen

Poly(D,L-lactide-co-glycolide) (PLGA) ist ein etabliertes bioabbaubares Polymer zur Herstellung von Nanopartikeln, die Zeitkontrolliert in wässriger Umgebung zersetzt werden und dabei einen darin verkapselten Arzneistoff freisetzen können. Ein am Markt sehr erfolgreiches ebenso formuliertes Beispiel ist Eligard^{®}, eine effektive Therapie zur Behandlung von Syptomen, die unter Prostata-Carcinomen vorkommen. Hierbei ist der Wirkstoff Leuprolid (ein Testosteron Inhibitor) in PLGA Nanopartikeln zur s.c. Injektion eingebettet.

### Beispiel VI:

Wie Beispiel VI zeigt, lassen sich mit Hilfe der levitronischen Pumpe PLGA basierte Nanopartikel mit und ohne Wirkstoffbeladung mit einem robusten Prozess über einen weiten Konzentrationsbereich herstellen. Als Beispielrezeptur wurde das von Evonik erhältliche RESOMER^{®} RG 502 H, ein Poly(D,L-lactide-co-glycolide), in variierendem Mischungsverhältnis zur wässrigen Phase (2%ig Polyvinylalkohol (PVA) Lösung) eingesetzt. Die PVA Lösung wurde als erste flüssige Phase vorgelegt, mit variierender Geschwindigkeit (Pump Speed) bewegt und die organische PLGA Lösung in Acetonitril als zweite flüssige Phase in variierender Geschwindigkeit hinzudosiert. Hierzu wurde RG 502 H in Acetonitril zuvor vollständig unter Rühren gelöst. Die Konzentration des PLGA in der organischen Phase betrug 10 mg/ml.

Die PLGA-Nanopartikel konnten auch erfolgreich mit einem schwer wasserlöslichen Modellwirkstoff, dem zur Behandlung von HIV zugelassenen Ritonavir, beladen werden. In den Verum Versuchen wurden zudem 10% bzw. 20% Ritonavir bezogen auf das PLGA zugesetzt.

Die resultierenden Partikel sind von ihrer Größe geeignet für eine Sterilfiltration, sowie von einer engen, die Qualität anzeigenden, Partikelgrößenverteilung. Es konnte gezeigt werden, dass für die beladenen Partikel ein in der pharmazeutischen Produktion erforderliches "Downstream processing" mittels Tangential Flow Filtration (TFF) zur Entfernung von nicht enkapsuliertem Wirkstoff und Restlösungsmittel, sowie Gefriertrocknung zum Erreichen einer erforderlichen Haltbarkeit, erfolgreich durchgeführt werden kann. Die erhaltenen Partikel weisen eine Verkapslungsrate (Encapsulation Efficacy) von bis zu 14.59 % auf und sind nach der Gefriertrocknung erfolgreich redispergierbar, wobei ähnliche Partikelgrößen und Partikelgrößenverteilung wie unmittelbar nach dem Herstellprozess erreicht werden.

Die Ergebnisse sind in Tabelle 6 niedergelegt.

### Beispielgruppe 4: Lipid Nanopartikel (LNP) als Arzneiträgersysteme für pharmazeutische Anwendungen, vorrangig mRNA

Seit der Zulassung eines ersten auf Lipid Nanopartikeln basierenden Präparats (Onpattro^{®}) durch die FDA im Jahr 2018 ist das Interesse an dieser Formulierungsstrategie gestiegen. Heute werden LNPs auch zur Formulierung von mRNA als aktive Bestandteile verwendet. Man kombiniert hierbei die Vorteile nanopartikulärer Formulierungen im allgemein (mechanischer Schutz des Wirkstoffs, längere Verweildauer im Organismus, bevorzugte Aufnahme in Tumorgewebe (EPR-Effekt)) mit spezifischen, durch die physikochemischen Eigenschaften der verwendeten Lipide bedingten Effekte. So lässt sich die chemisch unbeständige und geladene mRNA mit einem Lipid Nanopartikel durch die Zellmembran transportieren, wo bei niedrigerem pH ionisierbare Lipide dafür sorgen, dass die mRNA Fracht abgeben und im weiteren Verlauf die in der mRNA enthaltenen Information in Proteinen translatiert werden können.

### Beispiel VII:

Wie Beispiel VII zeigt, lassen sich mit Hilfe der levitronischen Pumpe Lipid Nanopartikel mit Poly-Adenosinmonophosphat (Poly A) als Surrogat für mRNA mit einem robusten Prozess herstellen.

Als Beispielrezeptur wurde eine Rezeptur verwendet, die DODMA (50mol%) als ionisierbares Lipid enthält und als weitere Bestandteile der Lipidfraktion DSPC, Cholesterol und PEG-DMG (10/38.5/1.5 mol%) bei einer kombinierten Lipidkonzentration von 15mM beinhaltet. Das Volumen der Lipidphase waren 20 ml. Das N/P Verhältnis lag in der Beispielrezeptur bei 3:1. Das Poly A wurde in 60 ml eines 5 mM Acetatpuffer bei pH 4.0 gelöst.

70 ml eines Phophatpuffer pH 7.4 USP wurden in den Behälter Vorlage gegeben und mit der levitronischen Pumpe bei 10.000rpm bewegt. Parallel wurde mittels zwei Kapillaren der Poly A enthaltende Acetatpuffer bei 15 ml/min und die Lipidlösung bei 5 ml/min injiziert. Beim Aufeinandertreffen der Lösungen im Bereich des Kapillarausgangs (siehe Skizze) wurden bei pH 4 Lipid Nanopartikel ausgebildet, die umgehend durch den Volumenstrom der levitronischen Pumpe mit dem Phosphatpuffer in Kontakt gebracht und durch dessen höhere Pufferkapazität in pH 7 überführt und somit stabilisiert wurden.

Mittels Gelelektrophorese (E-Gel^{®} EX invitrogen Agarose 1%) erhaltene Ergebnisse zeigen, dass das Poly A erfolgreich in den Lipid Nanopartikeln gebunden wurde, in der Injektionstasche des Gels verbleibt und hier ein starkes Signal erzeugt, während freies Poly A in das Gel hinein laufen konnte und eine typische Verteilung zeigt.

Die Ergebnisse sind in Tabelle 7 niedergelegt.

### Beispiel VIII:

Als Beispielrezeptur wurde eine Rezeptur verwendet, die DODMA (50mol%) als ionisierbares Lipid enthält und als weitere Bestandteile der Lipidfraktion DSPC, Cholesterol und PEG-DMG (10/38.5/1.5 mol%) bei einer kombinierten Lipidkonzentration von 15mM beinhalted. Das Volumen der Lipidphase waren 37,5 ml. Das N/P Verhältnis lag in der Beispielrezeptur bei 3:1. Das Poly A wurde in 112,5 ml eines 5 mM Acetatpuffer bei pH 4.0 gelöst und in den Behälter Vorlage gegeben und mit der levitronischen Pumpe bei 14.000rpm bewegt. Parallel wurde mittels einer Kapillare die Lipidlösung bei 5 ml/min injiziert. Beim Aufeinandertreffen der Lösungen im Bereich des Kapillarausgangs wurden bei pH 4 Lipid Nanopartikel ausgebildet. Die Partikellösung wurde aus der Vorrichtung entnommen und extern durch Zugabe von Phophatpuffer pH 7.4 USP auf pH 7 eingestellt und die Partikel somit stabilisiert.

Die Ergebnisse sind in Tabelle 8 niedergelegt.

### Beispielgruppe 5: Polyplexe als Arzneiträgersysteme für pharmazeutische Anwendungen, vorrangig mRNA

Ähnlich wie die Lipid Nanopartikel (LNPs) können auch Polyplexe, bspw. aus Polyethylenimin und (m)RNA oder DNA verwendet werden um diese Stoffe über eine non-virale Abgabe an ihren intrazellulären Wirkort zu bringen. Dabei können Polyplexe die Stabilität der wirksamen Bestandteile entscheidend erhöhen. Ladungsunterschiede zwischen dem in leicht saurer Lösung kationisch vorliegenden Polyethylenimin und den anionischen Phosphatresten der (m)RNA oder DNA bedingen die Ausbildung der Polyplexe.

### Beispiel IX:

80 ml RNase freies Wasser werden in der Vorlage des Geräts vorgelegt und bei 10.000 rpm zirkuliert (erste flüssige Phase). 33,5 ml einer 100µg/ml Poly A Lösung in RNase freiem Wasser werden unter Rühren hergestellt und dient als dritte flüssige Phase. Diese Phase wird bei 5 ml/min über eine Kapillare injiziert. Aus einer Stammlösung bestehend aus 10g/L Polyethylenimin werden 2.944 ml mit 3ml eines 1M Acetatpuffer und 29.056 ml Wasser gemischt (zweite flüssige Phase). Die daraus resultierende N/P Verhältnis liegt bei 15. Diese Mischung wird über eine weitere Kapillare bei 5ml/min injiziert.

Die Ergebnisse sind in Tabelle 9 niedergelegt.

### Beispiel X:

In Beispiel X werden identische Mengen von Poly A und Polyethylenimin wie in Beispiel IX eingesetzt, nur dass in diesem Beispiel die Poly A Lösung verdünnter in größerem Volumen (115 ml) in die erste Vorlage eingebracht und bei 10.000 rpm zirkuliert wird. Die bereits in Beispiel IX beschriebene Polyethylenimin-Lösung (35 ml) wird über eine Kapillare bei 5ml/min injiziert.

Die Ergebnisse sind in Tabelle 10 niedergelegt.

Aus der Zusammenschau der Beispiele IX und X erkennt der Fachmann, die gute Skalierbarkeit des mit der Vorrichtung betriebenen Verfahrens, da Poly A in Beispiel X in viel geringerer Konzentration über die erste flüssige Phase dosiert wurde als in Beispiel IX, wo Poly A über die dritte Phase eingetragen wurde.

**Tabelle 1: Ergebnisse Beispiel I**

| **Versuch Nummer** | **Konzentration Gesamtlipide (EPC-COL) in EtOH** | **Gesamtvolume im System [ml]** | **PumpenGeschwindigkeit [rpm]** | **Zugaberate [ml/min]** | **Probenahmezeit [min] (nach vollständiger Zugabe** | **Zav (backscatter)** | **PDI** |
|---|---|---|---|---|---|---|---|
| 21836/21 | 200mg/ml | 777.5 ml (700ml Ammoniumsulfat + 77.5 ml EPC-Col in EtOH) | 14.000 | 10 ml/ min | 10 | 153.2 | 0.309 |
| | | | | | 40 | 150.0 | 0.254 |
| | | | | | 70 | 147.5 | 0.246 |
| | | | | | 100 | 144.3 | 0.253 |
| 21836/29_2 | 200 mg/ml | 183.2 ml (150 ml Ammoniumsulfat + 33.2 ml EPC-Col in EtOH) | 14.000 | 5 ml /min | 5 | 155.6 | 0.216 |
| | | | | | 30 | 156.2 | 0.219 |
| | | | | | | 156.8 | 0.233 |
| | | | | | | 156.0 | 0.239 |

**Tabelle 2: Ergebnisse Beispiel II**

| **Versuch Nummer** | **Konzentration Gesamtlipide (EPC-COL) in EtOH** | **Gesamtvolume im System [ml]** | **PumpenGeschwindigkeit [rpm]** | **Probenahmezeit [min] (nach vollständiger Zugabe** | **Zav (backscatter)** | **PDI** |
|---|---|---|---|---|---|---|
| 21836/20 | 400 mg/ml | 500 ml (450 ml Ammoniumsulfat + 50 ml EPC-Col in EtOH) | 10.000 | 10 | 549.9 | 0.705 |
| | | | | 90 | 512.4 | 0.708 |
| 21836/24 | 400 mg/ml | 777.5 ml (700ml Ammoniumsulfat + 77.5 ml EPC-Col in EtOH) | 14.000 | 30 | 422.4 | 0.710 |
| | | | | | 398.5 | 0.785 |
| 21836/26 | 400 mg/ml | 777.5 ml (700ml Ammoniumsulfat + 77.5 ml EPC-Col in EtOH) | 8.200 | 30 | 1001 | 0.582 |
| | | | | | 973.1 | 0.838 |

**Tabelle 3: Ergebnisse Beispiel III**

| **Versuch Nummer** | **Konzentration Gesamtlipide (EPC-COL) in EtOH** | **Verhältniswässrige: Organische Phase** | **Gesamtvolume im System [ml]** | **Pumpen-Geschwind igkeit[rpm]** | **Rate der Zugabe der organischen Lösung [ml/min]** | **Kapillar Typ / Distanz zur Pumpe** | **Zav (backsc atter)** | **PDI** |
|---|---|---|---|---|---|---|---|---|
| 21836/33_1 | 15.2 mg/ml | 3:1 | 160 ml [120 ml wässrige Phase / 40 ml organische Phase] | 14.000 | 10 ml /min | Rohr ext Durchmesser 3.25mm / 20 mm | 108.4 | 0.136 |
| | | | | | | | 106.5 | 0.097 |
| 21836/33_2 | 15.2 mg/ml | 6:1 | 141 ml [120 ml wässrige Phase / 21 ml organische Phase] | 14.000 | 10 ml /min | Rohr ext Durchmesser 3.25mm / 20 mm | 92.67 | 0.144 |
| | | | | | | | 91.53 | 0.113 |
| 21836/33_3 | 15.2 mg/ml | 9:1 | 150 ml [135 ml wässrigePhase / 15 ml organische Phase] | 14.000 | 10 ml /min | Rohr ext Durchmesser 3.25mm / 20 mm | 92.81 | 0.129 |
| | | | | | | | 88.88 | 0.159 |
| 21836/33_4 | 15.2 mg/ml | 9:1 | 150 ml [135 ml wässrige Phase / 15 ml organische Phase] | 5.000 | 10 ml /min | Rohr ext Durchmesser 3.25mm / 20 mm | 90.75 | 0.134 |
| | | | | | | | 89.37 | 0.110 |
| 21836/33_5 | 15.2 mg/ml | 9:1 | 150 ml [135 ml wässrige Phase / 15 ml organische Phase] | 14.000 | 10 ml /min | Feine Kapillare / 20 mm | 81.12 | 0.160 |
| | | | | | | | 80.78 | 0.175 |
| 21836/33_6 | 15.2 mg/ml | 9:1 | 150 ml [135 ml wässrige Phase / 15 ml organische Phase] | 14.000 | 5 ml /min | Feine Kapillare / 20 mm | 81.56 | 0.202 |
| | | | | | | | 79.85 | 0.188 |

**Tabelle 4: Ergebnisse Beispiel IV**

| **Versuch Nummer** | **Konzentration Gesamtlipide (EPC-COL) in EtOH** | **Gesamtvolume im System [g]** | **PumpenGeschwindigkeit [rpm]** | **Rate der Zugabe der organischen Lösung [ml/min]** | **Kapillarabstand zur Pumpe** | **Zav (backscatter)** | **PDI** |
|---|---|---|---|---|---|---|---|
| 21836/30_1 | 386.90 mg/ml [38.69%] | 795 g [582.56g wässrige Phase / 212.24g organische Phase] | 14.000 | 5 ml /min | 20 mm | 238.3 | 0.216 |
| 21836/30_2 | 386.90 mg/ml [38.69%] | 795 g [582.56g wässrige Phase / 212.24g organische Phase] | 14.000 | 15 ml /min | 20 mm | 235.5 | 0.146 |
| 21836/30_3 | 386.90 mg/ml [38.69%] | 795 g [582.56g wässrige Phase / 212.24g organische Phase] | 14.000 | 25 ml /min | 20 mm | 224.7 | 0.173 |
| 21836/30_4 | 386.90 mg/ml [38.69%] | 795 g [582.56g wässrige Phase / 212.24g organische Phase] | 14.000 | 35 ml /min | 20 mm | 217.6 | 0.157 |
| 21836/30_5 | 386.90 mg/ml [38.69%] | 795 g [582.56g wässrige Phase / 212.24g organische Phase] | 5.000 | 15 ml /min | 20 mm | 314.4 | 0.286 |
| 21836/30_6 | 386.90 mg/ml [38.69%] | 795 g [582.56g wässrige Phase / 212.24g organische Phase] | 10.000 | 15 ml /min | 20 mm | 267.7 | 0.156 |
| 21836/30_7 | 386.90 mg/ml [38.69%] | 795 g [582.56g wässrige Phase / 212.24g organische Phase] | 14.000 | 15 ml /min | 20 mm | 234.9 | 0.125 |
| 21836/30_8 | 386.90 mg/ml [38.69%] | 795 g [582.56g wässrige Phase / 212.24g organische Phase] | 14.000 + 90 min im Kreis fahren nach Abschluss der Zugabe | 15 ml /min | 20 mm | 216.3 | 0.156 |
| 21836/30_9 | 386.90 mg/ml [38.69%] | 795 g [582.56g wässrige Phase / 212.24g organische Phase] | 14.000 | 15 ml /min | 10 mm | 238.6 | 0.232 |
| 21836/30_10 | 386.90 mg/ml [38.69%] | 795 g [582.56g wässrige Phase / 212.24g organische Phase] | 14.000 | 15 ml /min | 20 mm | 237.7 | 0.138 |
| 21836/30_11 | 386.90 mg/ml [38.69%] | 795 g [582.56g wässrige Phase / 212.24g organische Phase] | 14.000 | 15 ml /min | 30 mm | 230.7 | 0.174 |

**Tabelle 5: Ergebnisse Beispiel V**

| **Versuch Nummer** | **Konzentration Gesamtlipide (EPC-COL) in EtOH** | **Gesamtvolume im System [g]** | **PumpenGeschwindigkeit [rpm]** | **Rate der Zugabe der organischen Lösung [ml/min]** | **Kapillarabstand zur Pumpe** | **Zav (backscatter)** | **PDI** |
|---|---|---|---|---|---|---|---|
| 21836/30_12 | 193.45 mg/ml [19.35%] | 795 g [582.56g wässrige Phase / 212.24g organische Phase] | 14.000 | 15 ml /min | 20 mm | 197.9 | 0.126 |

**Tabelle 6: Ergebnisse Beispiel VI**

| **Versuch Nummer** | **Organische Phase** | **Vessel / Chargengröße** | **Kapillare** | **Pumpen-Geschwindigkeit[rpm]** | **Rate der Zugabe [ml/min]** | **Zav (backscatter)** | **PDI** |
|---|---|---|---|---|---|---|---|
| 21836/30 | ACN / 10 mg/ml / 77.8 ml | Stahl Tank / 777.8 ml nach Zugabe der org. Phase | Breit (3.25mm ext Durchmesser) | 14.000 | 10 ml /min | 101.3 | 0.200 |
| | | | | | | 100.2 | 0.197 |
| 21836/31_1 | ACN / 10 mg/ml / 15 ml | Plexiglas Tank / 150 ml nach Zugabe der org. Phase | Fein | 14.000 | 5 ml /min | 117.6 | 0.199 |
| | | | | | | 116.6 | 0.202 |
| 21836/31_2 | ACN / 10 mg/ml / 15 ml | Plexiglas tank / 150 ml nach Zugabe der org. Phase | Breit (3.25mm ext Durchmesser) | 10.000 | 10 ml /min | 88.43 | 0.213 |
| | | | | | | 87.77 | 0.202 |
| 21836/31_3 | ACN / 10 mg/ml / 15 ml | Plexiglas tank / 150 ml nach Zugabe der org. Phase | Breit (3.25mm ext Durchmesser) | 5.000 | 10 ml /min | 106.0 | 0.164 |
| | | | | | | 105.2 | 0.170 |
| 21836/31_4 | ACN / 10 mg/ml / 15 ml | Plexiglas tank / 150 ml nach Zugabe der org. Phase | Breit (3.25mm ext Durchmesser) | 2.500 | 10 ml /min | 99.70 | 0.214 |
| | | | | | | 98.41 | 0.204 |
| 21836/32_1 | ACN / 10 mg/ml / 15 ml | Plexiglas tank / 150 ml nach Zugabe der org. Phase | Breit (3.25mm ext Durchmesser) | 7.500 | 10 ml /min | 92.83 | 0.194 |
| | | | | | | 91.79 | 0.160 |
| 21836/32_2 | ACN / 10 mg/ml / 15 ml | Plexiglas tank / 150 ml nach Zugabe der org. Phase | Breit (3.25mm ext Durchmesser) | 10.000 | Bolus (15ml in 15 sek) | 102.6 | 0.205 |
| | | | | | | 102.1 | 0.197 |
| 21836/32_3 | ACN / 10 mg/ml / 15 ml | Plexiglas tank / 150 ml nach Zugabe der org. Phase | Breit (3.25mm ext Durchmesser) | 10.000 | Langsam (2.5 ml/min) | 105.4 | 0.170 |
| | | | | | | 103.7 | 0.177 |
| 1836/34_1 | ACN / 10 mg/ml / 15 ml + 1 % Ritonavir | Plexiglas tank / 150 ml nach Zugabe der org. Phase | Breit (3.25mm ext Durchmesser) | 10.000 | 10 ml /min | 98.06 | 0.251 |
| | | | | | | 96.94 | 0.239 |
| 1836/34_1 | ACN / 10 mg/ml / 15 ml + 2 % Ritonavir | Plexiglas tank / 150 ml nach Zugabe der org. Phase | Breit (3.25mm ext Durchmesser) | 10.000 | 10 ml /min | 102.0 | 0.272 |
| | | | | | | 98.06 | 0.199 |

**Tabelle 7: Ergebnisse Beispiel VII**

| **Versuchsnummer** | **Konzentration Gesamtlipide in EtOH** | **Gesamtvolume im System [g]** | **PumpenGeschwindigkeit [rpm]** | **Rate der Zugabe der organischen Lösung [ml/min** | **Kapillar Typ / Distanz zur Pumpe** | **Zav (backscatter)** | **PDI** |
|---|---|---|---|---|---|---|---|
| 21836/36_1 | 15 mM (thereof 7.5mM DODMA) | 150 ml 70 ml phosphate buffer pH 7.4 initially as outer phase in levitronic pump. 60 ml acetate buffer containing 17.4 mg Poly A added via capillary 20 ml lipid solution added via capillary | 10.000 | 15 ml / min for acetate buffer with Poly A via syringe pump 5 ml / min for organic lipid phase via peristraltic pump | Dual capillary (20 mm over levitronic pump head) | 5 min after addition: | |
| | | | | | | 104.6 | 0.207 |
| | | | | | | 101.9 | 0.216 |
| | | | | | | 30 min after addition: | |
| | | | | | | 97.86 | 0.197 |
| | | | | | | 95.91 | 0.200 |

**Tabelle 8: Ergebnisse Beispiel VIII**

| **Versuchsnummer** | **Konzentration Gesamtlipide in EtOH** | **Gesamtvolume im System [g]** | **PumpenGeschwindigkeit [rpm]** | **Rate der Zugabe der organischen Lösung [ml/min** | **Kapillar Typ / Distanz zur Pumpe** | **Zav (backscatter)** | **PDI** |
|---|---|---|---|---|---|---|---|
| 21836/37 | 15 mM (thereof 7.5mM DODMA) | 150 ml 112.5 ml actetate buffer pH 4.0 initially as outer phase in levitronic pump. 37.5 ml lipid solution added via capillary | 14.000 | 5 ml / min for organic lipid phase via syringe pump | single capillary (20 mm over levitronic pump head) | 5 min after addition: | |
| | | | | | | 122.2 | 0.367 |
| | | | | | | 115.6 | 0.340 |
| | | | | | | 30 min after addition: | |
| | | | | | | 135.4 | 0.292 |
| | | | | | | 135.0 | 0.289 |
| | | | | | | After adjustment to pH 7: | |
| | | | | | | 115.1 | 0.258 |
| | | | | | | 112.7 | 0.244 |

**Tabelle 9: Ergebnisse Beispiel IX**

| **Versuchsnummer** | **N:P Verhältnis** | **Gesamtvolume im System [g]** | **PumpenGeschwindigkeit [rpm]** | **Rate der Zugabe der organischen Lösung [ml/min** | **Kapillar Typ / Distanz zur Pumpe** | **Zav (backscatter)** | **PDI** |
|---|---|---|---|---|---|---|---|
| 21836/38 | 15 | 150 ml | 10.000 | 5 ml / min für Phasen über Kapillare | Doppelkappillare 20 mm über Pumpen kopf | 5 min nach Zudosierung: | |
| | | • 80 ml RNase freies Wasser als erste Phase in Pumpenumlauf | | | | | |
| | | | | | | 46.06 | 0.434 |
| | | | | | | 47.25 | 0.403 |
| | | • 35 ml wässrige Poly A Lösung über Kapillare | | | | 30 min nach Zudosierung: | |
| | | • 35 ml PEI Lösung in 20mM Acetat Puffer über Kapillare | | | | 60.41 | 0.396 |
| | | | | | | 58.79 | 0.422 |

**Tabelle 10: Ergebnisse Beispiel X**

| **Versuchsnummer** | **N:P Verhältnis** | **Gesamtvolume im System [g]** | **PumpenGeschwindigkeit [rpm]** | **Rate der Zugabe der organischen Lösung [ml/min** | **Kapillar Typ / Distanz zur Pumpe** | **Zav (backscatter)** | **PDI** |
|---|---|---|---|---|---|---|---|
| 21836/39 | 15 | 150 ml 115 ml wässrige Poly A Lösung als erste Phase über Umlauf 35 ml PEI Lösung in 20mM Actetat Puffer über Kapillare dosiert | 10.000 | 5 ml / min | Einzelkapillare (20 mm über Pumpenkopf) | 5 min nach Zudosierung: | |
| | | | | | | 41.51 | 0.336 |
| | | | | | | 43.27 | 0.318 |
| | | | | | | 30 min nach Zudosierung: | |
| | | | | | | 102.4 | 0.224 |
| | | | | | | 59.99 | 0.325 |

### Bezugszeichenliste:

- 0: Vorrichtung
- 1: erstes Vorlagegefäß
- 2: zweites Vorlagegefäß
- 3: Wirkelement
- 4: erste Zuleitung
- 5: zweite Zuleitung
- 6: Pumpe
- 7: Antrieb
- 7+: Drehfeld
- 8: Ablauf
- 8+: Umlauf
- 9: Entnahmearmatur
- 10: Gestell
- 11: Konus
- 12: erste Rohrleitung
- 13: Schliff
- 14: zweite Rohrleitung
- 15: dritte Rohrleitung
- 16: Innenwandung
- 17: Gehäuse
- 18: Läufer
- 111: Dorn
- 1111: Vorlagearmatur

## Patentansprüche

1. Vorrichtung für die Herstellung von Nanocarriern und/oder Nanoformulierungen, umfassend:
a) ein erstes Vorlagegefäß zur Aufnahme einer ersten flüssigen Phase;
b) ein zweites Vorlagegefäß zur Aufnahme einer zweiten flüssigen Phase;
c) ein Wirkelement zum Bereitstellen einer zumindest zweiphasigen Mischung durch Mischen der ersten flüssigen Phase mit der zweiten flüssigen Phase;
d) einen ersten Zulauf, über den das erste Vorlagegefäß mit dem Wirkelement fluidleitend verbunden ist;
e) einen zweiten Zulauf, über den das zweite Vorlagegefäß mit dem Wirkelement fluidleitend verbunden ist;
f) ein Sammelgefäß zur Aufnahme der Mischung;
g) einen Ablauf, über den das Wirkelement mit dem Sammelgefäß fluidleitend verbunden ist;
h) eine Pumpe, die in den Ablauf dergestalt eingegliedert ist, dass die Mischung vermittels der Pumpe von dem Wirkelement über den Ablauf in das Sammelgefäß förderbar ist;
**dadurch gekennzeichnet**,
i) dass der erste Zulauf und der zweite Zulauf zumindest auf einem Vertikalabschnitt vertikal ausgerichtet sind;
j) dass auf dem Vertikalabschnitt der zweite Zulauf innerhalb des ersten Zulaufs geführt ist;
k) und dass auf dem Vertikalabschnitt erste Zulauf oder der zweite Zulauf oder beide Zuläufe axial verschiebbar ausgeführt sind, dergestalt, dass die axiale Position des zweiten Zulaufs gegenüber dem ersten Zulauf veränderlich ist.

2. Vorrichtung nach Anspruch 1, **dadurch gekennzeichnet, dass** der erste Zulauf innerhalb des Vertikalabschnitts aus einer ersten linearen Rohrleitung gebildet wird, dass der zweite Zulauf innerhalb des Vertikalabschnitts aus einer zweiten linearen Rohrleitung gebildet wird, und dass die erste lineare Rohrleitung und die zweite lineare Rohrleitung auf dem Vertikalabschnitt koaxial verlaufen.

3. Vorrichtung nach Anspruch 1, weiter umfassend ein drittes Vorlagegefäß zur Aufnahme einer dritten flüssigen Phase sowie einen dritten Zulauf, über den das dritte Vorlagegefäß mit dem Wirkelement fluidleitend verbunden ist, wobei das Wirkelement zum Bereitstellen einer zumindest dreiphasigen Mischung durch Mischen der ersten flüssigen Phase mit der zweiten flüssigen Phase und der dritten flüssigen Phase eingerichtet ist, und wobei der erste Zulauf innerhalb des Vertikalabschnitts aus einer ersten linearen Rohrleitung gebildet ist, der zweite Zulauf innerhalb des Vertikalabschnitts aus einer zweiten linearen Rohrleitung gebildet ist und der dritte Zulauf innerhalb des Vertikalabschnitts aus einer dritten linearen Rohrleitung gebildet ist, **dadurch gekennzeichnet,**
a) **dass** der dritte Zulauf zumindest auf dem Vertikalabschnitt vertikal ausgerichtet ist;
b) **dass** auf dem Vertikalabschnitt der dritte Zulauf innerhalb des ersten Zulaufs geführt ist;
c) **dass** der dritte Zulauf auf dem Vertikalabschnitt axial verschiebbar ausgeführt ist, dergestalt, dass die axiale Position des dritten Zulaufs gegenüber dem ersten Zulauf veränderlich ist.

4. Vorrichtung nach Anspruch 3, **dadurch gekennzeichnet, dass** auf dem Vertikalabschnitt der dritte Zulauf innerhalb des zweiten Zulaufs geführt ist, insbesondere, dass die erste lineare Rohrleitung und die zweite lineare Rohrleitung und die dritte lineare Rohrleitung auf dem Vertikalabschnitt koaxial verlaufen.

5. Vorrichtung nach einem der Ansprüche 1 bis 4, **dadurch gekennzeichnet, dass** die Pumpe als eine Kreiselpumpe ausgeführt ist, welche ein Gehäuse und ein im Gehäuse um eine Drehachse drehbar gelagerten Läufer aufweist, welcher über einen Antrieb drehend antreibbar ist.

6. Vorrichtung nach Anspruch 5, **dadurch gekennzeichnet, dass** die Drehachse des Läufers vertikal ausgerichtet ist, und dass ein horizontaler Abschnitt des Ablaufs in der Drehebene des Läufers verläuft, wobei die Drehachse des Läufers vorzugsweise koaxial zur Achse des ersten Zulaufs im Bereich des Vertikalabschnitts ausgerichtet ist.

7. Vorrichtung nach einem der Ansprüche 5 oder 6, **dadurch gekennzeichnet, dass** der Läufer im Gehäuse magnetisch gelagert ist und dass es sich bei dem Antrieb um ein Drehfeld handelt, welches Leistungsübertragung von Antrieb auf Läufer frei von mechanischem Kontakt gestattet.

8. Vorrichtung nach einem der Ansprüche 1 bis 7, **dadurch gekennzeichnet, dass** das Sammelgefäß und das erste Vorlagegefäß identisch sind, und dass der Ablauf als ein Umlauf ausgeführt ist.

9. Vorrichtung nach einem der Ansprüche 1 bis 8, **dadurch gekennzeichnet, dass** der Förderstrom der Pumpe veränderlich ist.

10. Vorrichtung nach einem der Ansprüche 1 bis 9, **gekennzeichnet durch** mindestens eine Dosiervorrichtung, die zur Dosierung der zweiten flüssigen Phase in das Wirkelement eingerichtet ist.

11. Vorrichtung nach einem der Ansprüche 3 bis 10, **gekennzeichnet durch** mindestens eine Dosiervorrichtung, die zur Dosierung der dritten flüssigen Phase in das Wirkelement eingerichtet ist.

12. Vorrichtung nach Anspruch 10 oder 11, **dadurch gekennzeichnet, dass** der Förderstrom einer Dosiervorrichtung veränderbar ist, insbesondere, dass der Förderstrom aller Dosiervorrichtungen veränderbar ist.

13. Verfahren zur Herstellung eines Nanocarriers und/oder einer Nanoformulierung mit den folgenden Schritten:
a) Bereitstellen einer Vorrichtung nach einem der Ansprüche 1 bis 12;
b) Bereitstellen einer ersten flüssigen Phase im ersten Vorlagegefäß, wobei die erste flüssige Phase ein erstes flüssiges Dispersionsmedium enthält;
c) Bereitstellen einer zweiten flüssigen Phase im zweiten Vorlagegefäß, wobei die zweite flüssige Phase ein zweites flüssiges Dispersionsmedium und mindestens eine Komponente enthält, welche ausgewählt ist aus der Gruppe bestehend aus Präkursor eines Nanocarriers, Präkursor eines Wirkstoffes, Wirkstoff;
d) Antreiben der Pumpe zur Etablierung einer Flüssigkeitsströmung aus dem ersten Vorlagegefäß über den ersten Zulauf in das Wirkelement und über den Ablauf in das Sammelgefäß;
e) Dosieren der zweiten flüssigen Phase über den zweiten Zulauf in das Wirkelement, wobei der Volumenstrom der zweiten flüssigen Phase in dem zweiten Zulauf kleiner ist als der Volumenstrom der Flüssigkeitsströmung in dem ersten Zulauf;
f) Mischen von erster flüssiger Phase und zweiter flüssiger Phase in dem Wirkelement, wobei eine Mischung enthaltend einen Nanocarrier und/oder eine Nanoformulierung erhalten wird;
g) Ansammeln der Mischung in dem Sammelgefäß;
h) Entnahme der Mischung aus der Vorrichtung;
i) Optional: Aufarbeiten der Mischung, insbesondere Abtrennen des Nanocarriers und/oder der Nanoformulierung aus der Mischung.

14. Verfahren nach Anspruch 13, bei dem eine Vorrichtung nach einem der Ansprüche 3 bis 12 bereitgestellt wird, mit den folgenden Schritten:
a) Bereitstellen einer ersten flüssigen Phase im ersten Vorlagegefäß, wobei die erste flüssige Phase ein erstes flüssiges Dispersionsmedium enthält und wobei der pH-Wert der ersten flüssigen Phase zwischen 6 und 8, vorzugsweise 7 beträgt;
b) Bereitstellen einer zweiten flüssigen Phase im zweiten Vorlagegefäß, wobei die zweite flüssige Phase ein zweites flüssiges Dispersionsmedium und mindestens einen Präkursor eines Nanocarriers und/oder eines Wirkstoffes enthält und wobei der pH-Wert der zweiten flüssigen Phase zwischen 3 und 5, vorzugsweise 4 beträgt;
c) Bereitstellen einer dritten flüssigen Phase im dritten Vorlagegefäß, wobei die dritte flüssige Phase ein drittes flüssiges Dispersionsmedium und mindestens eine weitere Komponente umfasst, wobei die weitere Komponente ausgewählt ist aus der Gruppe bestehend aus Präkursor eines Nanocarriers, Wirkstoff, Präkursor eines Wirkstoffes;
d) Antreiben der Pumpe zur Etablierung einer Flüssigkeitsströmung aus dem ersten Vorlagegefäß über den ersten Zulauf in das Wirkelement und über den Ablauf in das Sammelgefäß;
e) Mischen von erster flüssiger Phase und zweiter flüssiger Phase und dritter flüssiger Phase durch Dosieren der dritten flüssigen Phase über den dritten Zulauf in das Wirkelement, wobei der Volumenstrom der dritten flüssigen Phase in dem dritten Zulauf kleiner ist als der Volumenstrom der Flüssigkeitsströmung in dem ersten Zulauf.

15. Verfahren nach Anspruch 13 oder 14, **dadurch gekennzeichnet, dass** es sich bei dem ersten Dispersionsmedium um Wasser handelt, und dass es sich bei dem zweiten Dispersionsmedium um eine organische Substanz handelt.

16. Verfahren nach Anspruch 15, **dadurch gekennzeichnet, dass** es sich bei der organischen Substanz um einen einwertigen oder mehrwertigen Alkohol handelt, wie beispielsweise Ethanol oder Glycerin.

17. Verfahren nach Anspruch 16, **dadurch gekennzeichnet, dass** es sich bei dem Präkursor des Nanocarriers um ein Phosphatidylcholin handelt, insbesondere um ein Phosphatidylcholin, welches aus Soja oder aus Ei gewonnen ist.

18. Verfahren nach Anspruch 15 oder 16 oder 17, **dadurch gekennzeichnet, dass** die erste flüssige Phase einen Puffer enthält, wobei der Puffer insbesondere ausgewählt ist aus der Gruppe bestehend aus Ammoniumsulfat, Acetat, Polyvinylalkohol, Phosphat, 2-(4-(2-Hydroxyethyl)-1-piperazinyl)-ethansulfonsäure.

19. Verfahren nach Anspruch 15 oder 16, **dadurch gekennzeichnet, dass** die erste flüssige Phase einen Puffer enthält, und dass die zweite flüssige Phase einen Wirkstoff oder einen Präkursor eines Wirkstoffes enthält; vorzugsweise, dass es sich bei dem Puffer um Polyvinylalkohol handelt, dass es sich bei der organischen Substanz um Acetonitril oder um Aceton oder um Dimethylsulfoxid oder um Ethylacetat handelt, und dass es sich bei dem Präkursor des Nanocarriers um ein Polylactid-co-Glycolid handelt.

20. Verfahren nach Anspruch 15 oder 16, **dadurch gekennzeichnet, dass** die erste flüssige Phase einen Puffer enthält, und dass die zweite flüssige Phase zwei Präkursor eines Nanocarriers enthält; vorzugweise, dass es sich bei dem Puffer um Ammoniumsulfat handelt, und dass es sich bei den beiden Präkursoren des Nanocarriers um Lipoid E PC und Cholesterol HP handelt.

21. Verfahren nach einem der Ansprüche 13 bis 20, bei dem eine Vorrichtung nach einem der Ansprüche 8 bis 12 bereitgestellt wird, **dadurch gekennzeichnet, dass** die erste flüssige Phase im Umlauf geführt wird, bevor oder während die zweite flüssige Phase dosiert wird.

22. Produkt, erhältlich nach einem Verfahren gemäß einem der Ansprüche 13 bis 21.

23. Produkt nach Anspruch 22, **dadurch gekennzeichnet, dass** es sich bei dem Produkt um einen Nanocarrier oder um eine Nanoformulierung handelt.

24. Produkt nach Anspruch 22 oder 23, mit einer mittleren Partikelgröße kleiner als 300 nm, vorzugsweise kleiner als 200 nm, besonders bevorzugt zwischen 40 nm und 140 nm, gemessen nach dem Prinzip der dynamischen Lichtstreuung mit dem Messgerät Zetasizer der Fa. Malvern Panalytical Ltd, GB, wobei der Winkel für Front und Bascatter auf 173° eingestellt ist.

25. Produkt nach Anspruch 22 oder 23 oder 24, mit einem Polydispersitätsindex zwischen 0.08 und 0.2, gemessen nach dem Prinzip der dynamischen Lichtstreuung mit dem Messgerät Zetasizer der Fa. Malvern Panalytical Ltd, GB wobei der Winkel für Front und Bascatter auf 173° eingestellt ist.

26. Produkt nach Anspruch 22 oder 23 oder 24, wobei es sich bei dem Produkt um eine Nanoformulierung handelt, welche eine nach der hier beschriebenen Messmethode gemessene Wirkstoffkonzentration aufweist, **dadurch gekennzeichnet, dass** die massenbezogenen Wirkstoffkonzentration von 1 ppm bis 50 % beträgt.
